(19) Europäisches Patentamt
European Patent Office
Office européen des brevets

(11) EP 3 428 815 A1

(12) EUROPEAN PATENT APPLICATION

(43) Date of publication:
**16.01.2019 Bulletin 2019/03**

(51) Int Cl.:
***G06F 19/16*** *(2019.01)* ***G06F 19/00*** *(2019.01)*

(21) Application number: **17305915.5**

(22) Date of filing: **11.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Institut Pasteur**
  **75724 Paris Cedex 15 (FR)**
- **Centre National de la Recherche Scientifique**
  **75794 Paris Cedex 16 (FR)**

(72) Inventors:
- **BOUVIER, Guillaume**
  **75014 Paris (FR)**
- **IZADI PRUNEYRE, Nadia**
  **75013 Paris (FR)**
- **SIMENEL, Catherine**
  **78220 Viroflay (FR)**
- **NILGES, Michael**
  **78960 Voisins le Bretonneux (FR)**

(74) Representative: **Desaix, Anne et al**
**Ernest Gutmann - Yves Plasseraud SAS**
**3, rue Auber**
**75009 Paris (FR)**

# (54) DOCKING METHOD BASED ON SATURATION TRANSFER DIFFERENCE NMR DATA, AND MEANS FOR ITS IMPLEMENTATION

(57) The invention relates to a method, especially a computer-implemented method, of identifying a collection of docking solutions representing a ligand-target interaction, which matches experimentally obtained structural assessment of said ligand-target interaction, said method involving recovery of data obtained by Saturation Transfer Difference - Nuclear Magnetic Resonance (STD-NMR) and recovery of computer-docked theoretical ligand-target complexes, and construction of a Self-Organizing Map (SOM). The identification of a collection of docking solutions is performed by applying a threshold value with respect to so-called X-type value(s). The invention also relates to a method of investigating or identifying the network of interactions, especially at the atomic level, involved in the binding between a ligand and its target, and to a computer program product or a computer readable medium, or a computer-displayed interface, as means for implementing the docking method of the invention.



Printed by Jouve, 75001 PARIS (FR)

## Description

The invention relates to a method, especially a computer-implemented method, of identifying a collection of docking solutions representing a ligand-target interaction, with the view to recover docking solutions that match experimentally obtained structural assessment of said ligand-target interaction.

The invention also relates to a method for investigating or identifying the network of interactions, especially at the atomic level, involved in the binding between a ligand and its target, and means to this end.

The invention also relates to a computer program product comprising software code adapted to cause a computer to perform the required step for implementing a method as disclosed herein, and specifically dedicated computer-displayed interface.

The invention may be used in the context of rational drug design and hit-to-lead optimization.

Detailed information on drug-target interaction is a prerequisite in academic and industrial programs of rational drug design and hit-to-lead optimization. More particularly, detailed structural information on the binding mode of a hit or a molecule of therapeutic interest and its target is a prerequisite in such programs[1,2]. According to common drug discovery experimentations, a hit molecule, also termed "ligand" herein, may emerge from target- or phenotype-based high throughput screening.

There is therefore a crucial need for rational drug design and hit-to-lead optimization to obtain pertinent structural information on drug-target interactions, including when target(s) has(have) been identified. Pertinent structural information is information enabling the determination of the interaction network, especially at the atomic level, between a hit and its target.

Non-experimental molecular docking methods enabling *in silico* generation of theoretical models representing the conformational interaction(s) between a ligand and its target are known, and can be used as virtual screening tools in structure-based discovery, but a true problem is to identify, amongst all the theoretical models that can be generated through such molecular docking methods, the theoretical model(s) that is(are) truly representative of a ligand-target interaction as found *in vitro, ex vivo,* or *in vivo.* Scoring functions provided by a docking software may be a limiting factor in virtual screening processes to find out all truly active ligands, for example because of a bad estimation of the ligand binding energies.

It has been proposed in WO 2009/032727 to combine a docking program with a scoring function that takes into account NMR Chemical Shift Perturbation data (NMR-CSP), i.e. changes in chemical shift of certain resonances in the NMR spectrum, to allow selection of docking poses portraying the hit in a biological, albeit soluble, context. The calculated score is used to generate a RMSD (root-mean-square deviation), which indicates how the theoretically generated pose matches the experimental assessment of a paradigm protein and paradigm ligand. However, NMR-CSP necessarily requires isotopic labelling of the target. NMR-CSP is based on the fact that the chemical shifts of atoms in the binding site of the target change when a ligand binds. In fact, the CSPs can be difficult to identify due to peak overlap and exchange between several conformations. Therefore, identification of amino acids involved in ligand binding can be impossible. The CSP can only be measured when the ligand is tightly bound to its target (such as when the dissociation constant (Kd) for the ligand-target binding is equal or superior to 5 $\mu$M). Such a high affinity is rarely observed for drug-target interactions. Furthermore, the method disclosed in WO 2009/032727 compares a single theoretical model to experimental data, and this does not take into account the intrinsic nature of biological active ingredients, which can freely move in their natural environment, i.e., which dynamically move and interact with targets.

In the course of drug design procedures, once a hit-target interaction has been validated, detailed structural information is needed to further guide hit-to-lead optimization. Nuclear Magnetic Resonance (NMR) enables one to determine a 3D structure of a hit-target complex and to gather crucial information about the binding mode at atomic resolution. It may however be preferred to gather information accurately representing the situation found in physiological or near physiological conditions.

The present therefore also addresses the need to obtain pertinent information approaching as close as possible the physiological conditions in which ligand-target interactions are naturally found, i.e., answers the general need to identify the structures actually representing the ligand-target binding mode *in vitro, ex vivo,* or *in vivo.*

The present invention is based on an original approach that combines NMR, molecular docking, and analysis based on machine learning to meaningfully characterize hit-target interactions at the atomic level, said approach being particularly suited for describing the reality of the physiological or near physiological context, and being, according to another advantageous aspect, also applicable in the context of NMR experiments on entire, whole, living cells.

More particularly, the invention makes use of data that can be gathered through Saturation Transfer Difference (STD) NMR (abbreviated STD-NMR herein).

STD-NMR is commonly used as a screening technique, and is particularly adapted to the characterization of the binding mode between small molecule ligands or hits and their target[3,4]. It is further appreciated that some, if not many, interesting targets are receptors that are embedded into the membrane and cannot be easily isolated and purified.

It is therefore another aspect of the invention, according to particular embodiments, to obtain pertinent structural

information on drug-target interactions that takes into account the fact that some proteins are membrane-bound proteins, or embedded into membranes, which impacts their naturally-occurring conformation and also makes them difficult to purify. In turn, this involves, when possible, time- and money- consuming steps of extraction, purification and stabilization of the target. In particular embodiments, the invention also addresses these problems.

A way to overcome this problem is to use STD experiments in the context of entire and living cells (*in cell*-STD), without any extraction or purification of the target. So far, *in cell*-STD has only been used in a small number of studies [4-6]. To the inventors' knowledge, the experiments reported herein are the first to use *in cell*-STD with whole bacterial cells. This experiment allows one to confirm the binding and to identify the atoms of the hit that are in contact with their cellular target. Moreover, this experiment affords inter-atomic distance information since the intensity of the NMR signal reflects the distance between a hit atom and its partner on the target side.

Atomic information obtained by NMR, especially STD-NMR, has to be interpreted in terms of structural data showing the network of atoms involved in the hit to target binding. The first step in getting these structural data consists in modeling of a large set of hit-target complexes by a standard small molecule-receptor docking approaches, as further described herein. In this step, only the molecular structures of the partners are used. The target structure can either be taken from a protein data bank or be built by molecular modeling. Next, the information experimentally obtained by NMR is included in the method of the invention: interacting atoms and the intensity of their NMR signals are directly used to drive a clustering algorithm based on an original machine learning approach that makes use of the Self-Organising Map algorithm[7]. This algorithm has been shown to be efficient for clustering a large amount of data, although its pertinence in the present context, where especially large amount of data is handled, was not predictable. The SOM algorithm allows the projection of multidimensional data onto a two dimensional grid space, in such a way that similar conformations are close together and dissimilar ones apart.

It is observed that the method of the invention takes into account NMR experimental data in a particular way, i.e., they are taken into account to drive the clustering performed subsequently to theoretical models data acquisition, and identifies an ensemble of hit poses in a particular way, from a very large set of solutions, on the basis of the constructed SOM. NMR data represent an average of conformations that are contributing to the dynamics of the molecular associations. The novel analysis method in turn also enables the identification of an ensemble of solutions that on average reproduce the NMR data, thereby accurately representing the biological reality of the molecular associations taking place *in vitro, ex vivo,* or *in vivo.*

Of note, the method of the invention identifies a whole ensemble of conformations of the drug bound to the target, which, together, account for the experimental data and are responsible for affinity and specificity of the drug for the target.

According to another aspect of particular embodiments, where *in cell*-STD data is used, the method of the invention does not require performance of NMR experiments requiring any extraction or purification of the target, which can be very large, can be part of a complex, or can be embedded in the membrane of a whole cell.

As detailed herein, and as a proof of concept, the inventors have illustrated the combined strategy of the method of the invention by studying the interaction of the anti-tuberculosis drug IPK317 disclosed in WO2011/113606, with its bacterial target. A chemical structure (A) of IPK317 with arbitrary proton numbering is depicted below. Chemical shifts, in ppm, assigned through NMR experiments are disclosed in the Table below (B).

A

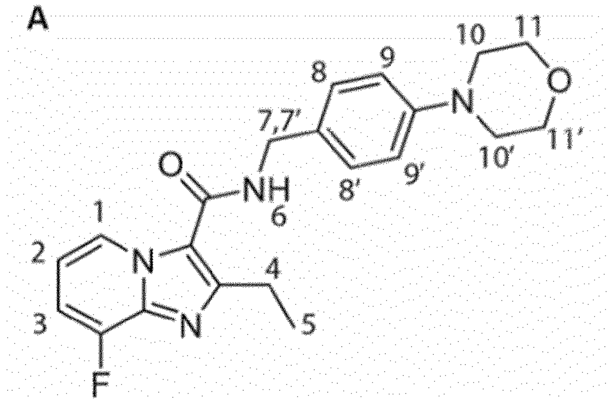

B

| Proton number | Chemical Shift (ppm) |
|---|---|
| 1 | 8.477 |
| 2 | 6.943 |
| 3 | 7.189 |
| 4 | 2.876 |
| 5 | 1.179 |
| 8, 8' | 7.346 |
| 9, 9' | 7.060 |
| 10, 10' | 3.113 |
| 11, 11' | 3.832 |

This drug belongs to a set of lead compounds with an imidazo (1,2-a) pyridine (IP) scaffold, which was identified by phenotype based high throughput screening[8]. By means of whole genome sequencing of spontaneous resistant

mutants, the putative target of IP drugs was identified as the cytochrome b (QcrB subunit) of the cytochrome bc1 complex, an essential component of the electron transport chain required for energy metabolism. QcrB is part of the cytochrome bcc-aa3 membrane supercomplex (QcrCAB-ctaCDEF), which makes its extraction and purification very challenging. Interestingly, this membrane complex is also the target of several other drugs, such as the antimalarial drug atovaquone. Using *in-cell* NMR, the inventors studied drug interactions with entire bacterial cells expressing wild type and mutant cytochrome bc1, and identified the chemical moieties involved in this interaction. Studying the interaction of IPK317 with entire bacterial cells expressing the *M. tuberculosis* QcrCAB (QcrCABMt) complex using the method of the invention, the inventors identified the chemical moieties involved in this interaction. The original analysis method of the invention led the inventors to obtain an ensemble of conformations that are in contact with the site of the spontaneous mutation leading to resistance, providing an independent validation of the approach. Of note, identifying an ensemble of drug poses proved to be important since a single conformation alone could not explain the experimental data.

The present invention therefore generally addresses the need for obtaining structural information on drug-target interactions that accurately depicts and takes into account the dynamics of drug-target interactions taking in physiological conditions. As a result, the invention provides outperforming tools that can be used for many modern drug development programs, in particular once the target has been identified or putatively defined. The approach disclosed herein promises to be highly advantageous for rational drug design and hit-to-lead optimization.

The invention therefore relates to a method, especially a partly or fully computer-implemented method, of identifying (to identify) a collection of docking solutions representing a studied ligand-target interaction, said collection in particular matching experimentally obtained structural assessment of said ligand-target interaction, said method comprising the several steps a. to e. described hereafter. It will be understood that the method of the invention is either a method for identifying a collection of docking solutions representing a studied ligand-target interaction, as described herein, or a method to identify a collection of docking solutions representing a studied ligand-target interaction, as described herein. The method of the invention preferably comprises steps a. to e. in the order corresponding to their alphabetical chronology, although some steps may be performed in a different order or concomitantly, without affecting the efficacy of the method of the invention. The skilled person will appreciate that some steps may be performed concomitantly or in a different order.

### *Experimental data*

In a first step a., the method involves recovery or provision of data corresponding to a set of several so-called **experimental STD NMR profiles,** or data corresponding to a single average experimental STD NMR profile resulting from the integration (average values of otherwise individualized values) of said several **experimental STD NMR profiles,** each profile consisting of a discrete collection of *n* STD ratios (*n* an integer), where each STD ratio is defined for each of the n (especially observable) protons of the ligand involved in the ligand-target interaction. A visual representation of an experimental STD NMR profile consisting of STD ratios is shown on Figure 4 (B).

Accordingly, in a particular embodiment, the several **experimental STD NMR profiles or** single average experimental STD NMR profile originate(s) from STD-NMR experiments separately carried out, and are(is) provided in a computer-readable form or as a computer-readable entry. According to another embodiment, the step of obtaining **STD NMR profiles** data is included in the method of the invention.

The skilled person is aware of the manner of setting the parameter of STD-NMR experiments so as to enable provision or recovery of **STD NMR profiles** of use for implementing the method described herein. He/she can readily adjust the parameters of the experiments carried out in an appropriate manner to obtain interpretable STD-NMR spectra. Examples are provided herein. Since several experiments are carried out, in part to provide an input that is representative of the biological reality of the molecular associations taking place *in vitro, ex vivo,* or *in vivo,* the skilled person is also in a position to optimize the parameters of the STD-NMR experiments so as to obtain measurable and reproducible signals.

According to a particular embodiment, the protons of the ligand involved in the ligand-target interaction are defined according to common practice in the art of NMR, especially on the basis of common NMR proton assignation protocols. Especially, the skilled person will readily understand that all protons giving rise to an observable and/or identifiable signal can or may be considered.

According to a particular embodiment, all protons of the ligand involved in the ligand-target interaction, which are observable by STD NMR, i.e., give rise to an interpretable signal, are considered for the purpose of the method of the invention. The skilled person readily knows how to assign protons on the basis of NMR spectra, and determine whether a proton is involved in the ligand-target interaction, on the basis of the experiments carried out. The skilled person can also eliminate proton(s) to be considered on purpose, although considering more protons generally adds valuable information to the method disclosed herein. For the purpose of the method of the invention, *n* protons of the ligand involved in the ligand-target interaction are retained. According to a particular embodiment, said *n* protons are all the observable protons defined using the recovered or input STD NMR spectra.

More particularly, in a preferred embodiment, in said first step a., the method involves recovery or provision of

data corresponding to a single average experimental STD NMR profile consisting of a discrete collection of *n* STD ratios (*n* an integer), where

- each STD ratio is defined for each of the n (especially observable) protons of the ligand involved in the ligand-target interaction, and
- in which for each proton *i* (*i* an integer, $1 \leq i \leq n$) an average experimental STD value $d_i$ is provided, said $d_i$ value averaging STD ratio values individually obtained through several STD NMR experiments, and
- each average experimental STD value $d_i$ being associated with a corresponding variance $\sigma_i^2$.

It will be understood that an average experimental STD NMR profile in step a. is obtained by averaging STD ratio values individually corresponding to several experimental STD NMR profiles.

Accordingly, average experimental STD NMR profile can be used if at least two **experimental STD NMR profiles** are available, which would in any case be preferred for assessing the experimental reproducibility.

According to a particular embodiment, at least 2 or 3, in particular between 2 and 5, especially 2, 3, 4 or 5 **experimental STD NMR profiles, corresponding to as much STD NMR experiments, are gathered to define** an average experimental STD NMR profile. According to a particular embodiment, at least 3 and up to 5 **experimental STD NMR profiles are recovered to define** an average experimental STD NMR profile. According to a particular embodiment, especially when *in-cell* STD NMR is performed, several, i.e., at least two, cell batches are used.

As defined herein, an **experimental STD NMR profile** is represented by a collection of several, especially *n* STD ratios, wherein each STD ratio is a ratio defined for each observable proton of the ligand involved in the ligand-target interaction, and wherein a STD ratio is defined as the ratio ($A_{STD}/A_0$) of the area of a proton signal in a STD NMR spectrum ($A_{STD}$) to that of the same signal in a reference spectrum (Ao), normalized to a maximum value set to 1 for the proton(s) with the highest STD signals.

According to a particular embodiment, especially as disclosed in the experimental section herein, the off-resonance spectrum is used as reference spectrum.

According to a particular embodiment, the reference spectrum is a 1H 1D NMR spectrum. According to this embodiment, the skilled person will appreciate that an 1H 1D NMR spectrum of the ligand alone used as a reference spectrum can suitably be recovered using the same relaxation time as that of the recovered STD spectra. Such an 1 H 1 D NMR spectrum can additionally be suitably used as a control to check the presence of to be expected signals.

Differently said, an **STD NMR profile** results from the comparison of a STD NMR spectrum obtained with a ligand in the presence of its target, with the off-resonance spectrum of same sample or an 1D NMR spectra of the ligand alone, i.e., a **STD NMR profile** corresponds to an "overall" ratio (considered over the whole profile) I(STD)/I(off res). This ratio is normalized to a maximum value set to 1 for the ligand proton that is the closest to the target in the ligand-target interaction.

The skilled person will readily understand that the proton(s) with the highest STD signal is(are) the proton(s) closest to the target in the ligand-target interaction.

According to a particular embodiment, the STD NMR used is STDD NMR.

Once data corresponding to a set of several **experimental STD NMR profiles** is retrieved, an average experimental STD value $d_i$ can be determined, said value $d_i$ being determined for each observed proton *i* (*i* an integer, $1 \leq i \leq n$). Value $d_i$ averages the STD ratios values individually obtained through the several STD NMR experiments considered, each average experimental STD value $d_i$ being associated with a corresponding variance $\sigma_i^2$.

Therefore, according to a particular embodiment, the data provided as an input to the method of the invention in step a. are average experimental STD values $d_i$ and their corresponding variance $\sigma_i^2$.

Alternatively, it is possible to gather and/or compute, within the method of the invention, and from **experimental STD NMR profiles** as defined herein, said average experimental STD value $d_i$ and corresponding variance $\sigma_i^2$.

Data defined in step a. can be provided to the method of the invention using input data files, especially data files encompassing the STD ratios values constitutive of the average **experimental STD NMR profiles or experimental STD NMR profiles, additionally or not with the corresponding average** $d_i$ values and their corresponding variance, e.g., in Excel files.

### *Theoretical data*

In a second step b., the method involves recovery or provision of so-called theoretical data, especially data generated by molecular docking or molecular dynamics simulation.

Accordingly, in a particular embodiment, the theoretical data is generated by molecular docking or molecular

dynamics simulations separately carried out, and are provided in a computer-readable form or as a computer-readable entry. According to another embodiment, the step of generating molecular docking or molecular dynamics simulation data is included in the method of the invention.

Two types of data are provided to the method of the invention in step b.: **computer-generated theoretical STD profiles** mimicking the experimental STD NMR profiles of step a., and cartesian coordinates of the ligand docked in several ligand-target poses, the latter being provided for the subsequent construction of a Self-Organizing Map (SOM), as described hereafter.

Accordingly, in a second step b., the method involves obtaining (i.e., recovering, providing, or generating), from *N* (*N* an integer) computer-docked ligand-target complexes (as a synonym: ligand-target poses), especially obtained by molecular docking or molecular dynamics simulation:

- A set of *N* **computer-generated theoretical STD profiles,** each profile consisting of a discrete collection of *n* theoretical STD ratios, where each theoretical STD ratio is defined for each of the *n* (especially corresponding to the experimentally observable) protons of the ligand determined to be involved in the ligand-target interaction, and
- A set of *N* Cartesian coordinates of the ligand docked in said ligand-target complexes (as a synonym: ligand-target poses).

The computer-generated theoretical STD profiles are an approximation of experimentally obtained STD NMR profiles, based on the most influencing factor for experimental data, i.e, the buriedness of the protons within the target. Accordingly, computer-generated theoretical STD profiles consisting of STD ratios can be visually represented like experimental STD NMR profiles, e.g., as shown on Figure 4 (B).

Therefore, prior to the provision of computer-generated theoretical STD profiles and Cartesian coordinates, computer-docked ligand-target complexes are obtained through suitable methods, which include molecular docking or molecular dynamics simulations. Examples are provided herein. From the retained computer-docked ligand-target complexes, one can extract the two types of data described above.

According to a particular embodiment, the *n* protons of the ligand determined to be involved in the ligand-target interaction are the same as those defined herein in the section above relative to STD NMR experiments, i.e., the *n* protons are defined with respect to the assignment retained for and from the experimental data used for implementation of the method of the invention.

It is observed that it is possible, at that point, when *N* **computer-generated theoretical STD profiles or their corresponding data are gathered,** to determine for each proton *i* in said profiles (*i* an integer, $1 \leq i \leq n$, preferably $i = n$ with observed protons that correspond to those observed experimentally) a theoretical STD ratio $x_i$, (*i* an integer, $1 \leq i \leq n$)), which can be used in subsequent calculations, if needed.

In particular, it is possible, at that point, to compute for each of the *N* theoretical STD profile a X-value according to the formula (a weighted least-square method):

$$\chi = \sqrt{\sum_{i=1}^{n} \frac{(x_i - d_i)^2}{\sigma_i^2}}$$

wherein $x_i$ is the theoretical STD ratio, as defined in step a., for each of the *n* protons, *i* ranging from 1 to *n,* and $d_i$ is the experimental STD value defined in step a. for each *n* proton,

$\sigma_i^2$ is the variance associated with the experimental STD value $d_i$.

The set of *N* cartesian coordinates of the ligand docked in ligand-target complexes is preferably recovered from the same molecular docking or molecular dynamics simulations enabling recovery of the **computer-generated theoretical STD profiles.** According to a particular embodiment, the set of *N* Cartesian coordinates is obtained from the same simulations as those enabling recovery of computer-generated theoretical STD profiles, in particular respectively corresponds to the same theoretical ligand-target poses used for providing both type of data.

The skilled person is aware of the manner of running molecular docking or molecular dynamics simulations according to guidance known in the art so as to enable provision or recovery of **computer-generated theoretical STD profiles** and Cartesian coordinates of use for implementing the method described herein. Further guidance and examples are provided herein.

According to a particular embodiment, N is at least 10000.

According to a particular embodiment, N ranges from 10000 to 105000. Further details are provided hereafter

regarding this parameter, which can be adjusted by the skilled person according to his/her knowledge.

According to the above, a **computer-generated theoretical STD profile** in step b. is represented by a collection of ratios, each ratio value being defined for each observable proton of the ligand involved in the ligand-target interaction as a so-called parameter B ("Buriedness"), which has been defined to approach the meaningfulness, at the physical level, of experimental STD ratios.

The calculation of parameters B requires the selection of the protons of the ligand and the protons of the target around the protons of the ligand, on the generated computer-docked ligand-target complexes used for determination of theoretical STD profiles and Cartesian coordinates.

According to a particular embodiment, the protons of the ligand are determined by comparison with and/or according to the protons of the ligand observable by 1H 1D NMR and/or STD NMR experiments, i.e., they are attributed to the docked ligand during the docking or molecular dynamics simulations procedures on the basis of the identification of the protons of the ligand observed experimentally in NMR experiments. NMR enables the attribution of a signal to proton(s) or group(s) of protons. This attribution can be reported on the chemical structure of the ligand used for computer models generation.

According to a particular embodiment, the protons of the target around the protons of the ligand are selected during docking or molecular dynamics simulations procedures, using a nearest neighbor search algorithm. The skilled person will appreciate that any conventional nearest neighbor search algorithm or a variant thereof can be used. Nearest neighbor search algorithms commonly enable the identification of the nearest neighbors of a query without calculating all the distances between neighbors. According to a particular embodiment, the nearest neighbor search algorithm is the KDTree search algorithm, with default parameters or appropriately adjusted parameters. The skilled person is aware of the manner of adjusting the search parameters for its purposes. According to a particular embodiment, the KDTree search algorithm is implemented as shown in the Examples.

As defined herein, parameter B can be computed as the number of protons of the target in a sphere of diameter chosen between 5.0 Å and 7.0 Å around an observed ligand proton, divided by the total number of (i.e., divided by the sum of all the) protons of the target encompassed within the (sum of the/all the) spheres centered at each proton of the ligand, said parameter B being normalized to a maximum value set to 1 for the most buried proton of the ligand within the studied ligand-target interaction. For instance, if a sphere of diameter 6 Å around an observed ligand proton is retained, parameter B is calculated as the number of protons of the target in a sphere of diameter 6.0 Å around an observed ligand proton, divided by the total number of protons of the target that are closer than 6.0 Å to any proton of the ligand, normalized to a maximum value set to 1 for the most buried proton of the ligand within the studied ligand-target interaction.

According to a particular embodiment, the diameter value retained for the spheres centered at each proton of the ligand depends upon, and corresponds to, the sphere diameter around the observed ligand proton. According to another particular embodiment, the diameter value retained for the spheres centered at each proton of the ligand can range, independently from the value retained for the sphere diameter around the observed ligand proton, between the values described herein.

The diameter of the sphere used can be selected amongst: 5.0, 6.0, 7.0 Å, and any value in between these values and within the range of 5.0 Å to 7.0 Å. According to a particular embodiment, the diameter of the spheres is 6.0 Å.

It will be understood that parameter B corresponds, at the physical level, to the buriedness of an observed ligand proton, i.e., the extent to which said proton is close to the target and/or is subjected to the influence of said target (considering the influence investigated by NMR). It is observed that the intensity of a STD NMR signal for an observed proton directly depends upon the number of protons in close (direct) vicinity with said observed proton. The influence of neighboring protons decreases by a factor of $1/r^6$ with the distance r between the observed proton and the neighboring proton. Therefore, the inventors reasoned that STD NMR ratio values could be approximated by calculating the parameter B value defined herein.

Data defined in step b. can be provided to the method of the invention using input data files, especially data files encompassing the STD ratios values constitutive of the **computer-generated theoretical STD profiles,** and Cartesian coordinates, as detailed above, e.g., in Excel files. According to a particular embodiment, X-values as defined above may also be provided.

***SOM Construction***

It is observed that the method of the invention permits that both the ligand and the target partners are flexible during the docking step. This strongly favors a realistic view of the ligand-target complex association, as found in physiological or near to physiological conditions, by increasing the authorized conformational degrees of freedom when taking into account possible ligand-target complex conformations. However, such a fully flexible docking procedure significantly increases the number of conformations to be generated. Therefore, identifying a relevant collection of docking solutions representing a studied ligand-target interaction would beneficiate from an appropriate reduction of the

high-dimensional data sets generated. In this respect, the present invention proposes an elegant, efficient and tailored solution.

In a third step c., the method of the invention encompasses constructing a Self-Organizing Map (SOM) having P (*P* an integer, $P \leq N$) nodes.

A SOM, also termed Kohonen's Self-Organizing Map, is a data visualization technique as well as a clustering technique, which reduces the dimensions of input data through the use of self-organizing neural networks. "Self-organizing" means that a SOM is trained using unsupervised learning to produce a low-dimensional (typically two-dimensional), discretized representation of the higher-dimensional input space of the training samples. This representation can typically take the form of a map. Map units, or neurons, usually form a two-dimensional lattice that can conveniently be represented, as such, in a more accessible form. Therefore, SOMs enable the mapping of high dimensional space input data onto a plane. However, while reducing the dimensionality of input data, SOMs also group similar data together, thereby enabling the display of similarities amongst input data. And finally, SOMs enable the creation of a network that stores information in such a way that topological relationships within the training set are maintained.

A SOM consists of components called nodes or neurons. Implementation of a SOM requires a training phase during which the map is built using input examples. In the end, the nodes of the SOM are allocated with weight vector(s) of the same dimension as used input data vectors, and with a position in the map space. Several distinct weight vectors in the training set can be allocated to a same node, whereas some nodes may remain void. Conversely, each input data of the training set is allocated to one unique node of the SOM. This node is defined as the node with the smallest Euclidean distance with the considered input vector. It's worth noticing that the Euclidean distance is generally used to compute distances to assign input vectors to the SOM nodes. However, one can define other metrics to the SOM, and this metric can be passed to the current implementation to be used instead of the default Euclidean distance. Reference is made to Daniela Digles and Gerhard F. Ecker, Self-Organizing Maps for In Silico Screening and Data Visualization, Mol. Inf. 2011, 30, 838 - 846 (DOI: 10.1002/minf.201100082) for further details. The skilled person is aware of the manner of constructing and training a SOM according to guidance known in the art so as to enable provision of a SOM of use for implementing the method described herein. Further guidance and examples are provided herein.

In the context of the present invention, training has for purpose to assign the Cartesian coordinates of the ligand docked in several ligand-target poses, as found in each theoretical model built by molecular docking or molecular dynamics simulation, to SOM map coordinates (nodes, in this context).

To each docking pose corresponds a unique set of Cartesian coordinates of the ligand. Allocating said set of Cartesian coordinates to a SOM node during training can be performed considering the smallest Euclidean distance between the set of Cartesian coordinates to be allocated to a node and a vector's node homogenous to the considered set of Cartesian coordinates (or other parameter(s)). Therefore, it is possible that several set of Cartesian coordinates are, in the end, allocated to a same node of the SOM.

Conversely, as explained above, some nodes of the SOM may remain void, i.e., not allocated with any input data.

Once all input Cartesian coordinates are assigned to nodes, it is possible to map within the SOM all type of data associated with said Cartesian coordinates. Such data can be the N input computer-generated theoretical models (e.g., their corresponding STD profiles), and any data that can be tracked back or forth with respect to said Cartesian coordinates, i.e., data that can be associated to a pose identified through its Cartesian coordinates. In the present context, mapping is a synonym for "making a correspondence between data in the input space (Cartesian coordinates) and data in the output space (correspondence to a position on the SOM map)". Use can be made of a correspondence table or any convenient type of data storage or storage method or correspondence method for this purpose.

Once the SOM is trained with the provided Cartesian coordinates, it is therefore possible to map within the SOM data associated with the computer generated poses corresponding to said Cartesian coordinates, such as the input computer-generated theoretical models, especially their corresponding **computer-generated theoretical STD profiles,** and/or data that can be generally defined as properties of the computer generated poses, such as X-values or $X_j$-values or $X_k$-values or $X_k$-profile curves according to any embodiment described herein, or Buriedness values (B parameter), Energy of the docked poses according to the definitions available in the art, number of H-bonds, distances between atoms within the docked poses...

All data that can be associated with the Cartesian coordinates allocated to defined nodes of the SOM through the training procedure can *a posteriori* (i.e., after SOM training and of course when said data to be allocated is made available) be projected onto a visual representation of the SOM map, resulting in a so-called 2D SOM map, a particular example of which is a U-matrix, as further detailed herein and in the literature available to the skilled person.

By "2D SOM map" or "2D SOM grid", which are used interchangeably herein, it is meant herein any type of visual representation commonly known and readily implementable by the skilled person based on his/her knowledge and the literature in the art, which is suitable for representing or vizualizing data contained in or associated to a SOM. For example, typical examples of "2D SOM maps" are described in Juha Vesanto, "SOM-based data visualization methods", Intelligent Data Analysis, Volume 3, Issue 2, 1999, Pages 111-126[19]. Particular and further examples of "2D SOM maps" include "distance matrices", as for example described in section 3.1.4 of Vesanto (page 117 lines 13-31),

which is incorporated herein by reference. Accordingly, according to a particular embodiment, a 2D SOM map is a representation that is a distance matrice, especially according to the provided reference. Depending upon the data to be displayed, outputting a 2D SOM map can be achieved as soon as the SOM has been trained (and once the data to be displayed is made available - the skilled person knows how to adjust the steps disclosed herein so that data to be displayed onto a 2D SOM map can be subsequently displayed). A 2D SOM map, as generally defined herein, enables the projection of data that can be associated to Cartesian coordinates as defined herein, in a two dimensional space. It will be understood that as long as the particular data that can be determined and gathered through the presently disclosed method is outputted and displayed using a common SOM visualization method, the skilled person can readily adapt the visualization method to meet his/her needs, especially for the purpose of interacting with the user of the method, in particular in the conclusion step.

According to a particular embodiment, a 2D SOM map displays X-type values as defined herein, especially $X_j$-values. According to another embodiment, an appropriate visualization output, especially for interaction with the method described herein, displays $X_k$-value(s).

Basically, in step c., of the method of the invention, a SOM having P (*P* an integer, *P* ≤ *N*) nodes is:

- trained with the set of *N* cartesian coordinates of the ligand of step b., and
- mapped with data gathered from the N computer-docked ligand-target complexes of step b., especially mapped at least with the N **computer-generated theoretical STD profiles** of step b., optionally with structures of the *N* computer-docked ligand-target complexes of step b.

At that point, and as explained above, it is possible to map the SOM, since training is complete, with all type of data that can be associated with the *N* computer-docked ligand-target complexes, the Cartesian coordinates of which have been used to train the SOM. For instance, according to a particular embodiment, it is possible to calculate or retrieve a X-value as defined above, which can be associated to a single, particular theoretical STD profile, and therefore a particular computer-docked ligand-target complex.

Any type of value can be assigned to the P nodes of the constructed SOM, especially, but not only, for representation purposes.

Assignation of values (mapping) to the P nodes of the SOM, enables reducing the data collected in the N space to a discretized P space.

Then, for each node P of the mapped SOM to which several theoretical STD profiles are assigned, a **mean theoretical STD profile** is defined using all the theoretical STD profiles assigned to said P node as a result of the mapping step. This **mean theoretical STD profile** can be further assigned to (mapped in) said node P of the SOM, or made available according to any suitable method, such as through a correspondence table or appropriate data storage, as known in the art. In the situation where a node P contains only one **theoretical STD profile,** said single profile is accordingly the mean theoretical STD profile. In the situation where no input data is allocated to a particular node P (void cell/node), no mean theoretical STD profile can be calculated. Such a node remains void.

Mean theoretical STD profiles are obtained (calculated) by computing mean values for all corresponding STD ratios of several theoretical STD profiles of a same node.

Upon completion of all these steps, up to P mean theoretical STD profiles can therefore mapped in the nodes of the constructed SOM, i.e., P nodes are filled with data when no empty node exist. In any event, upon completion of these steps, one mean theoretical STD profile is assigned to each non-empty node of the SOM.

In addition, step c. of the method described herein can encompass (such as in step b.), a step of calculating for each node P of the mapped SOM, a $X_j$-value according to the formula:

$$\chi_j = \sqrt{\sum_{i=1}^{n} \frac{(MeR_{theo\,i} - d_i)^2}{\sigma_i^2}}$$

wherein

*j* is an integer between 1 and *P*, and
*n* is the number of observed protons (and consequently the number of discrete STD ratios found in within a STD profile (each profile consisting of a discrete collection of *n* STD ratios)), and
$MeR_{theoi}$ is a mean theoretical STD ratio for each of the *n* protons of the **mean theoretical STD profile,** and
*i* ranges from 1 to *n, and*
$d_i$ is the experimental STD value defined in step a. for each *n* proton,

$\sigma_i^2$ is the variance associated with the experimental STD value $d_i$

According to a particular embodiment, $X_j$-values associated with mean theoretical STD profiles can be used for graphical presentation, such as X-values defined above can be (such values can be projected on a 2D SOM map/grid). $X_j$-values are however representative of the mean theoretical STD ratios of several poses allocated to a single node of the SOM, whereas X-values are representative of a single pose.

According to a particular embodiment, P ranges from 10 to 10000, in particular from 25 to 10000. Further details are provided hereafter regarding this parameter, which can be adjusted by the skilled person according to his/her knowledge.

Once training of the SOM is complete, it is possible to visualize the SOM, for example using a distance matrix as further detailed herein, in particular a U-matrix (Unified distance matrix) representation of the SOM, which is conventionally used to visualize the distances between the nodes of SOM, or a similar representation such as the projection of another data type on the frame provided by a U-matrix, or direct visualization of data associated with the nodes of the SOM within a so-called 2D SOM grid. The skilled person can readily choose, on the basis of the intended purpose, the best representation model to display.

A U-matrix can provide an helpful, albeit limited, visual representation of a SOM when one tries to find clusters in the input data without having any a priori information about the clusters. A U-matrix shows the relations between the neighboring neurons of a SOM. To display a U-matrix, one only needs the data related to the coordinates of the ligand (the Cartesian coordinates), which are assigned to the neurons of the SOM once training has been completed. A U-matrix displays U-values for each cell of the SOM, calculated as follows: the U-value of cell *i* corresponds to the mean of Euclidean distances between the input vectors of cell *i* and that of the 8 neighboring cells.

The U-matrix of Figure 3a displays U-values in Angstroms.

It is also possible to project any data type on a 2D SOM grid (map), let it be on a representation corresponding to a U-matrix or not. Any type of data that can be associated to the nodes of the SOM can be displayed, in particular by projection on a so-called 2D map. For example, Figure 3b shows a projection of $X_j$-values.

According to a particular embodiment, the method of the invention encompasses the projection of any data that can be associated to the nodes of the constructed SOM on a 2D SOM map and/or a distance matrix, in particular a U-matrix, especially X-values or $X_j$-values as defined herein. Displaying such X-type values on a graph readily enables the user of the method to apply a threshold to this data, which can in particular be determined interactively. $X_k$-value(s) can also be displayed, in order to enable the user to apply a threshold to this data, which can in particular be determined interactively. According to a particular embodiment however, $X_k$-values are displayed within a $X_k$-profile as disclosed herein.

**Data-driven clustering**

According to a particular embodiment, the method described herein enables the identification of a collection of docking solutions, which matches the experimental structural assessment of the ligand-target interaction of step a., using a data-driven clustering approach. In this embodiment, $X_k$-values as described below are calculated and used to drive the clustering, thereby appropriately integrating a reference, if not a comparison, to NMR experimental data.

To this end, a fourth step d. may be performed, through which the method described herein encompasses clustering the *P* mean theoretical STD profiles of the nodes of the SOM, through:

i. Ordering the nodes of the SOM, especially through a flooding algorithm applied to the result of a minimum spanning tree algorithm, and returning an ordered list of up to *P* nodes (or an ordered list of P nodes if the used ordering algorithm is developed until the last available iteration corresponding to a node P), each node being associated with a mean theoretical STD profile, the ordering of the nodes being obtained through a flooding algorithm, and

ii. For each node *k* (*k* an integer, $1 \leq k \leq P$) of the ordered list of up to *P* nodes, providing (computing) an **average theoretical STD profile** averaging the mean theoretical STD profiles of all the preceding *P* nodes, including the instant node *k,* said **average theoretical STD profile** consisting of a discrete collection of *n* average theoretical STD ratios $AvR_{theo}$, where each average theoretical STD ratio ($AvR_{theo\ i}$ (*i* an integer, $1 \leq i \leq n$)) is defined for each of the *n* (especially corresponding to the experimentally observable) protons of the ligand determined to be involved in the ligand-target interaction, and

iii. Calculating for each node *k* of the ordered list of up to *P* nodes, an $X_k$-value according to the formula (a weighted least-square method):

$$\chi_k = \sqrt{\sum_{i=1}^{n} \frac{(AvR_{theoi} - d_i)^2}{\sigma^2}}$$

wherein

k is an integer between 1 and P, and
n is the number of observed protons and consequently the number of discrete STD ratios found in within a STD profile (each profile consisting of a discrete collection of *n* STD ratios), and
$AvR_{theoi}$ is an average theoretical STD ratio for each of the *n* protons of the average **theoretical STD profile,** and
i ranges from 1 to *n, and*
$d_i$ is the experimental STD value defined in step a. for each *n* proton,
$\sigma_i^2$ is the variance associated with the experimental STD value $d_i$
iv. Optionally providing, in particular returning or displaying, a **$X_k$-profile curve** displaying the computed $X_k$-value(s) in ordinate and the ordered list of up to P nodes in abscissa.

Regarding point i. and according to a particular embodiment, the ordering the nodes of the SOM can be achieved using a so-called flooding algorithm, as known in the art and as such readily available for implementation to the skilled person. An exemplary flooding algorithm readily available to the skilled person is cited and described in Bouvier, G. et al, Bioinformatics 2015, 31, 1490 (reference [18] herein). It will be understood that the skilled person can readily adapt such an algorithm to his/her needs when implementing the method described herein.

Regarding point i. and according to another particular embodiment the ordering of the nodes can be performed through a flooding algorithm applied to the result of a minimum spanning tree algorithm.

The skilled person will readily understand that any conventional minimum spanning tree algorithm, as known and reported in the literature, can be used.

For example, the SOM map can be, and in a particular embodiment is, first depicted by building a minimum spanning tree, using conventional and appropriate algorithms, in particular a Kruskal algorithm, as illustrated in the Examples section herein. The result of a minimum spanning tree algorithm applied to a SOM map consists in one or a series of graphs representing several possible ordered list of neurons. A flooding algorithm can, and in a particular embodiment is, then applied to these graphs. A particular example of flooding algorithm is the Dijkstra algorithm as illustrated in the Examples section herein. Applying such a flooding algorithm to such graphs enables to determine and return an ordered list of neurons.

According to a particular embodiment as described in the Examples section herein, the result of the Kruskal minimum spanning tree algorithm applied to a SOM map consists in one or a series of graphs representing several possible ordered list of neurons. Applying the Dijkstra algorithm to said graphs enables to determine and return an ordered list of neurons. The skilled person readily knows that the Dijkstra algorithm is an algorithm for finding the shortest paths between nodes in a graph, the implementation of which can follow variations that are all within his/her skills. According to the embodiment of this paragraph, it will be understood that the flooding algorithm is an implementation of a Dijkstra algorithm, applied to a two dimensional grid.

According to a particular embodiment, the flooding algorithm is applied to the result of a minimum spanning tree algorithm.

According to a particular implementation, the flooding algorithm can be applied to any cell (node) *i* of the SOM. Starting from cell (node) *i,* the nearest neighbor is selected, defining the new current cell (node), then the algorithm is iteratively applied to the new cell (node) to finally cover all the map. The nearest neighbor search is based on the minimum spanning tree previously computed.

Regarding point ii., the provision of an **average theoretical STD profile** averaging the mean theoretical STD profiles of all the preceding *P* nodes follows the procedure already describe above in the section relative to step c. of the method of the invention, when a **mean theoretical STD profile** is defined. Average theoretical STD profiles are accordingly obtained by computing mean values for all corresponding STD ratios of the several mean theoretical STD profiles of all the preceding *P* nodes.

Regarding point iii., it is appreciated that the specifically defined $X_k$-value is used to drive the clustering performed on the basis of an ordered list of nodes of a SOM, by appropriately integrating a reference, if not a comparison, to NMR experimental data. Absolute $X_k$-values (or absolute $X_j$- or X-values discussed above) can range from 0 to 1.5, and are in themselves a good descriptor of the match between theoretical STD profiles and experimental STD profiles: a small value indicates a good match between the considered theoretical model(s) (or collection of theoretical models) and the experimental one(s), the 0 value being a perfect match. An example of $X_k$-values display is illustrated in Figure 4a.

Regarding point iv., "providing" means that the method of the invention at least calculates or stores on a computer the corresponding data, and/or returns the drawn $X_k$-profile curve, or data permitting the visualization of such a curve, as an output. According to a particular embodiment, the method includes the step of displaying the resulting curve, and/or the storage of said curve or data permitting its visualization, on appropriate support, e.g., in the memory of a computer, let it be the computer used for implementing the method of the invention or a distinct and/or distant computer, or on a portable support. A schematic $X_k$-profile curve is depicted in Figure 1. According to a particular embodiment, the method is arranged to that component(s) can display the resulting curve, and/or store said curve or data so as to enable its visualization, on appropriate support, e.g., in the memory of a computer, let it be the computer used for implementing the method of the invention or a distinct and/or distant computer, or on a portable support.

Generally, unless differently specified, the expression "to return" or "returning" used herein can be understood as a synonym for "to provide" or "providing".

***Conclusion***

In a final step, which may be numbered step d. or step e. depending on the fact that the data-driven clustering step discussed above is performed or not, and in order to return (provide) a collection of docking solutions representing the studied ligand-target interaction, in particular a collection which matches the experimental structural assessment of said ligand-target interaction provided in step a., the method of the invention encompasses the identification of a collection of docking solutions by:

- especially when the data-driven clustering detailed above is not performed, but not exclusively, applying a **threshold value** with respect to the $X_j$-values associated with the nodes of the SOM then calculated in step c., wherein the threshold value is pre-determined and/or interactively determined, especially interactively determined through a computer-displayed interface as described herein, or through interaction with a 2D SOM grid displaying $X_j$-values, and the collection of docking solutions consists of all the computer-generated mean theoretical STD profiles of the nodes of the SOM, which are associated with a computed $X_j$-value that is equal or below said threshold, **and**/or
- when the data-driven clustering detailed above is performed, applying a **threshold value** to the $X_k$-profile curve obtained in step d. iv., wherein the threshold value is determined as corresponding to a **local** minimum value $X_{min\text{-}loc}$ on the $X_k$-profile curve, the collection of docking solutions consisting of all the computer-generated mean theoretical STD profiles of the nodes of the SOM, which result, after implementation of step d., in an $X_k$-value on the $X_k$-profile curve that is equal or below the local minimum value $X_{min\text{-}loc}$, or
- when the data-driven clustering detailed above is performed, applying a **threshold value** to the $X_k$-profile curve obtained in step d. iv., wherein the threshold value corresponds to a **global** minimum value $X_{min}$ on the $X_k$-profile curve, the collection of docking solutions consisting of all the computer-generated mean theoretical STD profiles of the nodes of the SOM, which result, after implementation of step d., in an $X_k$-value on the $X_k$-profile curve that is equal or below the global minimum value $X_{min}$,

and optionally returning (providing) the collection of identified docking solutions.

It will be understood that when the data-driven clustering is not performed, calculation of $X_j$-values in step c. is required (not optional) in order to provide X-type values data for the concluding step. Conversely, when data-driven clustering is performed, the possibility to apply a threshold value to a $X_k$-profile curve is made available.

It will be understood that the step of data-driven clustering described above enables the provision of a $X_k$-profile curve, which can be suitably used to determine a threshold to apply to return (provide) a collection of docking solutions. According to a particular embodiment, when the conclusion is reached by applying a **threshold value** to a $X_k$-profile curve that corresponds to global minimum value $X_{min}$ of the $X_k$-profile curve, the method of the invention automatically identifies the collection of docking solutions on the basis of the data associated to the nodes of the SOM during implementation of any embodiment of the method described herein.

According to another embodiment, when a conclusion is reached by applying a **threshold value** to a $X_k$-profile curve, and where said threshold value corresponds to local minimum value $X_{min\text{-}loc}$ of the $X_k$-profile curve, this value can be predetermined and/or interactively defined by the user of the method upon consideration of the $X_k$-profile curve that is provided as an intermediate step, or can be provided by an appropriate computer algorithm enabling the identification of one or several local minimum value $X_{min\text{-}loc}$ (with a further choice let to the user, if several local minimum value $X_{min\text{-}loc}$ are recovered).

For example, the threshold value to apply to a $X_k$-profile curve can be defined considering the amount of ligand-complex structures recovered in a cluster as a function of the applied threshold. The skilled person will understand that the number of ligand-complex structures recovered in a cluster can be displayed as an output on an appropriate interface. The skilled person may also adjust the threshold value to apply depending upon the number of ligand-complex structures he/she accepts to recover.

Conversely or in addition to the above, especially when an interface is used to represent the $X_j$-values associated with a SOM, or $X_k$-profile curve resulting from the implementation of the method described herein, when a conclusion is reached by applying a threshold value that is interactively determined, interaction with the computer can be performed through a computer-displayed interface as described herein, or through an interface enabling visualization of a 2D SOM grid or an appropriate data visualization display, said interface or grid displaying for example $X_j$-values or a $X_k$-profile curve as defined herein.

The skilled person can readily define which type of graphical representation is well suited for its purpose, especially for defining a threshold value. Particular examples of graphical representations are provided in the experimental section herein (e.g., in Figures 3 and 4, for illustration purposes). As a particular example, data can be further projected on a particular type of graphical representation as a substructure. For example, the display of X-type values, especially $X_j$-values, on a 2D SOM map can follow a frame obtained by displaying a U-matrix, i.e., X-type values, especially $X_j$-values, can be further projected on a 2D SOM map, the basis of which is a U-matrix.

Considering only conventional U-matrices, the data displayed is obtained on the basis of the Cartesian coordinates stored in the nodes of the SOM only. Therefore, a U-matrix representation only enables the visualization of the structural homogeneity of a cluster displayed after application of U-value threshold. Applying a threshold on a U-matrix (a U-value threshold) only enables the definition of clusters, without taking into account experimental data.

Conversely, the fact of using X-type values, which correlate experimental and theoretical data, and thresholds of the same type, enables the definition of clusters taking into account experimental data.

The conclusion can also be reached by applying a threshold value that is pre-determined, which does not exclude the possibility that the selected threshold value corresponds to a local or global minimum of a $X_k$-profile curve, although not required.

Threshold values are accordingly of the "X-value type".

As defined herein, "X-value types" encompass values calculated on the basis of any one of the equations described herein. Separated equations are provided herein for X-values, $X_j$-values and $X_k$-values, because said values can be calculated at different steps of the method described herein, and reflects in some cases the integration of differently aggregated data (A X-value can be calculated for a single theoretical model compared to experimental data, $X_j$-values and $X_k$-values can be defined in the context of mean or average theoretical STD profiles, respectively). All these types of X-value however have the same significance with respect to their interpretation, and are part of the present invention.

As explained above, in absolute, X-type values including $X_k$-values are in themselves good descriptors of the match between "theoretical" STD profiles (obtained through computer methods, as disclosed herein) and "experimental" STD profiles (obtained via experimentation, as disclosed herein). A small value indicates a good match between the considered theoretical model(s) (or collection of theoretical models) and the experimental one(s), the 0 value being a perfect match. In theory absolute X-type values are positive values for which the upper limit for a X-type value is highly related to the quality of the experimental data. A X-value or X-type value ranging from 0 to 1 means that the error between the "theoretical" STD profiles and the "experimental" STD profiles is inferior to the experimental error. Therefore, in practice a model with an associated X-value in the range of 1 to 1.5 can be meaningful for determining collection of docking solutions to be identified though a method as described herein.

Accordingly, when the threshold value is pre-determined, the X-type threshold value can range from 0 to 1.5, with all intermediate values encompassed. According to a particular embodiment, a threshold value can have the value: 0, 0.1, 0.2, 0.3, 0.4, 0.5, 0.6, 0.7, 0.8, 0.9, 1.0, 1.1, 1.2, 1.3, 1.4, 1.5 or any value in between. A threshold value can range from 0 to 0.5, or 0.1 to 0.4, or 0.1 to 0.3, or 0.1 to 0.2, or 0.2 to 0.3, or 0.2 to 0.4, or 0.2 to 0.5, or range between any combination of boundaries as disclosed herein. A threshold value can correspond to a local or global $X_k$-value on the $X_k$-profile curve defined herein. A threshold value can correspond to the average of all $X_k$-values associated with the nodes of a considered SOM.

Returning (providing) the collection of identified docking solution means that the method provides data corresponding to, or enabling the identification of, all the computer-generated mean theoretical STD profiles (associated with the nodes of the SOM) that enables meeting the condition set above. The returned (provided) data can be directly visualized, especially on a 2D SOM map as defined herein, or provided as an output, i.e., a computer-readable file or text file.

According to a particular embodiment, the method includes the step of displaying the returned collection of docking solutions, and/or the storage of said data permitting its visualization, on appropriate support, e.g., in the memory of a computer, let it be the computer used for implementing the method of the invention or a distinct and/or distant computer, or on a portable support.

The collection of docking solutions identified by the method disclosed herein is also termed "cluster" or "best cluster" herein.

The invention therefore relates, according to a particular aspect, to a computer-implemented method of identifying (to identify) a collection of docking solutions representing a ligand-target interaction, which matches experimentally obtained structural assessment of said ligand-target interaction, said method comprising (the steps of):

a. Obtaining, from several Saturation Transfer Difference - Nuclear Magnetic Resonance (STD-NMR) experiments investigating the studied ligand-target interaction, an average experimental STD NMR profile consisting of a discrete collection of *n* STD ratios (*n* an integer), where each STD ratio is defined for each of the *n* (especially observable) protons of the ligand involved in the ligand-target interaction, in which for each proton *i* (*i* an integer, $1 \leq i \leq n$) an average experimental STD value $d_i$ is provided, each average experimental STD value $d_i$ being associated with a corresponding variance $\sigma_i^2$ ;

b. Obtaining, from *N* (*N* an integer) computer-docked ligand-target complexes, especially obtained by molecular docking or molecular dynamics simulation:

- A set of N computer-generated theoretical STD profiles, each profile consisting of a discrete collection of n theoretical STD ratios, where each theoretical STD ratio is defined for each of the n (especially corresponding to the experimentally observable) protons of the ligand determined to be involved in the ligand-target interaction, and;
- A set of *N* cartesian coordinates of the ligand docked in said ligand-target complexes;

c. Constructing a self-organizing map (SOM) having P (*P* an integer, $P \leq N$) nodes, where:

- The SOM is trained with the set of *N* cartesian coordinates of the ligand of step b., and
- The SOM is mapped with the N **computer-generated theoretical STD** profiles of step b., and
- For each node P of the mapped SOM, a **mean theoretical STD profile** is calculated using the theoretical STD profiles of the preceding step allocated to said node P, and
- Optionally, calculating for each node P of the mapped SOM, a $X_j$-value according to the formula:

$$\chi_j = \sqrt{\sum_{i=1}^{n} \frac{(MeR_{theoi} - d_i)^2}{\sigma_i^2}}$$

wherein

*j* is an integer between 1 and *P*, and
*n* is the number of observed protons, and
MeRtheoi i is a mean theoretical STD ratio for each of the *n* protons of the mean **theoretical STD profile,** and
*i* ranges from 1 to *n, and*
$d_i$ is the experimental STD value defined in step a. for each *n* proton,
$\sigma_i^2$ is the variance associated with the experimental STD value $d_i$

d. Optionally, clustering the *P* **mean theoretical STD profiles** of the SOM, through:

i. Ordering the nodes of the SOM, and returning an ordered list of up to *P* nodes, the ordering of the nodes being obtained through a flooding algorithm, and
ii. For each node *k* (*k* an integer, $1 \leq k \leq P$) of the ordered list of up to *P* nodes, computing an **average theoretical STD profile** averaging the mean theoretical STD profiles of all the preceding P nodes, including the instant node *k,* said **average theoretical STD profile** consisting of a discrete collection of n average theoretical STD ratios $AvR_{theo}$, where each average theoretical STD ratio ($AvR_{theoi}$ (*i* an integer, $1 \leq i \leq n$)) is defined for each of the *n* protons of the ligand determined to be involved in the ligand-target interaction, and
iii. Calculating for each node *k* of the ordered list of up to *P* nodes, an $X_k$-value according to the formula (a weighted least-square method):

$$\chi_k = \sqrt{\sum_{i=1}^{n} \frac{(AvR_{theoi} - d_i)^2}{\sigma_i^2}}$$

wherein

k is an integer between 1 and P, and

n is the number of observed protons and consequently the number of discrete STD ratios found in within a STD profile (each profile consisting of a discrete collection of *n* STD ratios), and

$AvR_{theoi}$ is an average theoretical STD ratio for each of the *n* protons of the average **theoretical STD profile,** and

*i* ranges from 1 to *n, and*

$d_i$ is the experimental STD value defined in step a. for each *n* proton,

$\sigma_i^2$ is the variance associated with the experimental STD value $d_i$

iv. Providing (returning) a **$X_k$-profile curve** displaying the computed $X_k$-value(s) in ordinate and the ordered list of up to *P nodes* in abscissa, and

e. Identifying a collection of docking solutions by either:

- especially when step d. is not performed, applying a **threshold value** with respect to the $X_j$-values associated with the nodes of the SOM calculated in step c., wherein the threshold value is pre-determined and/or interactively determined, especially interactively determined through a computer-displayed interface according to any embodiment described herein, or through interaction with a 2D SOM grid displaying $X_j$-values, and the collection of docking solutions consists of all the computer-generated mean theoretical STD profiles of the nodes of the SOM, which are associated with a computed $X_j$-value that is equal or below said threshold, and/or
- when step d. is performed, applying a **threshold value** to the $X_k$-profile curve obtained in step d. iv., wherein the threshold value is determined as corresponding to a **local** minimum value $X_{min-loc}$ on the $X_k$-profile curve, the collection of docking solutions consisting of all the computer-generated mean theoretical STD profiles of the nodes of the SOM, which result, after implementation of step d., in an $X_k$-value on the $X_k$-profile curve that is equal or below the local minimum value $X_{min-loc}$, or
- when step d. is performed, applying a **threshold value** to the $X_k$-profile curve obtained in step d. iv., wherein the threshold value corresponds to a **global** minimum value $X_{min}$ on the $X_k$-profile curve, the collection of docking solutions consisting of all the computer-generated mean theoretical STD profiles of the nodes of the SOM, which result, after implementation of step d., in an $X_k$-value on the $X_k$-profile curve that is equal or below the global minimum value $X_{min}$,

and optionally providing (returning) the collection of identified docking solutions.

As described above in the Section "Theoretical data", provision of computer-generated theoretical STD profiles and Cartesian coordinates in step b. requires obtaining computer-docked ligand-target complexes through suitable methods, which include molecular docking or molecular dynamics simulations.

According to a particular embodiment, when molecular docking is used, the following protocol can be followed:

- provision or generation of one or several conformations of the molecular structure of the studied target, especially between 500 and 1500 conformations, especially generating an ensemble of conformations representing the studied ligand-target interaction site, and then
- generation, for each conformation of the target retrieved from the above step, of several poses of the studied ligand-target complex, by docking several poses, especially between 20 and 70 poses, of a conformational molecular model of the ligand onto a single target conformation, using a computer-implemented docking program.

The number of conformations of the molecular structure of the studied target can readily be adjusted by the skilled person. In order to determine the number of conformations of the ligand to generate, it is possible to calculate the rate of generation of structural clusters of the target as a function of the simulation time, and fix a maximal pertinent number of conformations to be generated when the generation rate hits a plateau. The skilled person will readily understand that the number of conformations to be used, especially in a pertinent manner, may depend on the studied target. According to a particular embodiment, between 500 and 1500 conformations can be generated. According to another embodiment however, it is possible not to taken into account the flexibility of the target, and only rely on one target conformation. In the Examples, 1000 conformations have been generated.

According to particular embodiments, 500, 600, 700, 800, 900, 1000, 1100, 1200, 1300, 1400, 1500 conformations, or any number in between, are generated.

The generation of several conformations of the molecular structure of the studied target can suitably be performed on the basis of a pre-existing conformational template model of the departure target molecular structure, as available to the skilled person, or a conformational template model build by a protein conformational sampling method. Any protein conformational sampling method available in the art is suitable. Without being limiting, examples of conformational sampling methods include molecular dynamics simulations, Monte Carlo simulations, Simulated annealing,

Replica exchange or adaptations of these methods. Any method suited for refinement and optimization of peptide and protein structures may accordingly be used. According to a particular embodiment, the Rosetta Relax program may be used.

According to a particular embodiment, care is taken to generate an ensemble of conformations more particularly representing the studied ligand-target interaction site.

The generation of several poses of the studied ligand-target complex, for each retained conformation of the target, can be conventionally be achieved by docking, i.e., using a computer-implemented docking program. Numerous docking programs are available to the skilled person. Any docking program may be used. In the Examples section herein, the inventors have used the Dock program. Such programs enable the docking of a conformational molecular model of a ligand onto a conformational molecular model of the target. The conformational molecular model(s) of the target is(are) the conformation(s) discussed above. A ligand model is docked in said target model(s), so as to generate several poses. The number of poses to generate can take into account the flexibility of the ligand and/or the volume of the pocket a ligand may target. A flexible ligand may require generation of more poses to realistically represent all the manners according to which it can interact with its target. According to a particular embodiment, between 20 and 70 poses can be generated. According to another embodiment however, in the Examples, 50 poses have been generated.

According to particular embodiments, 20, 30, 40, 50, 60, 70 poses, or any number in between, are generated.

According to a particular embodiment, when molecular dynamics simulations are used instead of molecular docking for obtaining theoretical computer-docked ligand-target complexes, several poses of the studied ligand-target complex are obtained by molecular dynamics simulation, especially on the basis of:

- a pre-existing conformational template model of the ligand-target complex structure, such as a template model based on a crystallographic structure of the ligand-target complex, or
- a conformational molecular model of the ligand-target complex structure, which is obtained via a computer-implemented docking program.

It will be understood that any manner of suitably providing several theoretical molecular models of the studied ligand-target complex can be used. Integration of more data may enable to provide a more representative picture of the conformational interaction possibilities within the studied ligand-target complex, at the theoretical level.

The method therefore involves retrieving several, especially N, generated molecular models of the studied ligand-target complex and obtaining their corresponding **computer-generated theoretical STD profiles, and also recovering the corresponding** Cartesian coordinates of the ligand docked in said ligand-target complexes.

The number N may therefore depend upon the manner of acquiring computer generated models. According to a particular embodiment, N can range between 10000 and 105000. In the Examples, 50000 theoretical models have been generated. According to particular embodiments, N is selected amongst: 10000, 20000, 30000, 40000, 50000, 60000, 70000, 80000, 90000, 100000 or any number in between, or can range between any one of these values taken as boundaries, although N values superior to 100000 or 105000 are not excluded. In fact, there is no material limit, especially computer limit to the amount of structures that can be clustered using the method described herein, since the calculation time evolves linearly with the number N of structures to cluster. According to a particular embodiment, N is at least 10000 (with no upper limit).

As described above in the Section "SOM construction", the SOM used for implementing the method of the invention can, according to particular embodiments:

- Have a size that is defined by a *l* per *r* array of nodes, with *l* ranging from 10 to 100 and *r* ranging from 10 to 100. According to a particular embodiment, the SOM has a node size of 50 per 50, which in turn fixes the P value discussed herein to 2500. It will be appreciated that the size of the SOM can be commonly adjusted by the skilled person, if required by performing several trials. A SOM having a *P* node size of 50 per 50 can be represented by a SOM map, especially a 2D SOM grid, having 2500 cells (also termed grids, or nodes, herein). According to particular embodiments, a 2D SOM grid representing a SOM of the invention as a size ranging from 100 to 10000 grids. Accordingly, the value of P discussed herein can range from 10 to 10000, although it will be appreciated that the P value, which depends upon the defined SOM size, may vary accordingly. In fact, every SOM iteration requires performing P calculations. The skilled person will appreciate that no adaptation difficulty is to be foreseen if P ranges between 25 (5x5) and 10000 (100x100), and/or
- Use for training purposes Cartesian coordinates of the ligand docked in the computer-generated complexes discussed herein, as detailed in the present description, especially uses N Cartesian coordinates and/or is mapped with at least the **computer-generated theoretical STD profiles** discussed herein, especially N **computer-generated theoretical STD profiles,** and/or
- Is trained with at least one training phase, in particular a first training phase with a number of iterations that is roughly equal to the number of input data used for training, i.e., about N iterations, in particular 50000 iterations in the case

of the experiments reported herein. According to a particular embodiment, the SOM is trained with two training phases, in particular the second training phase has a number of iterations that is two times the number of iterations used in the first training phase. According to a particular embodiment, the number of iterations in the second training phase is 100 000, as reported herein. Increasing the intensity of the learning in the second training phase may ensure convergence of the SOM. The skilled person can readily adjust the number of training phases and the number of iterations according to his/her knowledge.

Other parameters that may be adjusted for SOM constructions include:

- The learning rate in each of the performed training phases. According to a particular embodiment, the learning rate is in the range of 0.5 to 0.25 for the first phase and 0.25 to 0 for the second phase, i.e., the learning rate is an exponentially decreasing learning rate. Specific values may be adjusted by the skilled person.
- The Gaussian radius used in each of the performed training phases. According to a particular embodiment, the Gaussian radius is from 6.25 (50/8) to 3.125 for the first phase and from 3.125 to 1.0 for the second phase, i.e., the Gaussian radius is an exponentially decreasing Gaussian radius. Specific values may be adjusted by the skilled person.

According to a particular embodiment, the parameters used for constructing and training the SOM are as shown in the Examples herein, especially regarding map size, number of training phases and number of iterations for the training phases, learning rate and radius used in said training phases.

According to a particular embodiment, the STD NMR experiments of step a. are *in cell*-STD NMR experiments. According to a more particular embodiment, the STD NMR experiments of step a. are *in cell*-STD NMR experiments carried out on whole bacterial cells.

According to a particular implementation of the method of the invention, the STD NMR experiments of step a. are performed and included within the steps of the method of the invention. According to this embodiment, the method of the invention includes carrying out STD NMR experiments and acquiring STD spectra subsequently used for determining experimental STD profiles and gathering of data as required by the method disclosed herein, according to any part of the present disclosure.

According to a particular embodiment, STD NMR experiments and STD NMR spectra acquisition are performed as shown in the Examples section herein. However, it will be understood that the skilled person can readily adjust the STD experiments parameters and experimental condition so as to obtain reproducible experiments, as discussed above, using his/her common knowledge.

According to a particular embodiment wherein *in cell*-STD NMR experiments are carried out, STD NMR spectra are acquired by a standard pulse sequence, with on- and off-resonance saturation frequencies of 0.0 and 35.0 ppm, by a train of 40 selective E-BURP-2 pulses of 50 ms each, separated by a 1 ms delay, with a total separation time of 2 s, optionally with the application of a Th1rho filter of 10 ms. According to a particular embodiment, the off-resonance spectrum is used as a reference spectrum.

The method of the invention is readily applicable to a wide range of ligands. According to particular embodiments, the ligand can be selected amongst: a peptide or oligopeptide, a nucleic acid molecule such as DNA or a RNA molecule, a metabolite, a carbohydrate or structural variant thereof -and a natural or synthetic chemical compound such as chemical small molecule drug (especially a candidate for drug discovery). Examples of synthetic chemical compounds (such as nicotine, caffeine...) or drugs are widely known in the art.

It will be understood that a chemical compound is a suitable ligand in the context of the present invention if the number and/or localization of its proton(s) giving rise to an interpretable signal by NMR provide sufficient information for studying the ligand-target association by NMR, especially STD NMR according to the present disclosure. For example, existence of only 10 protons giving rise to interpretable NMR signals, when these proton(s) can be found in several places of the ligand so as to provide enough information regarding interaction with the target, would provide sufficient information for implementation of the method of the invention.

The skilled person will understand that suitable ligands should remain stable during the NMR experiments carried out, or stable during a period of time sufficient to gather NMR data, which can be appreciated by the skilled person. When *in cell*-STD NMR is contemplated, and according to a particular embodiment, ligand(s) are soluble under physiological conditions.

The method of the invention is readily applicable to protein target(s). Target(s) can be selected amongst: a protein, especially a receptor, more particularly a membrane-embedded receptor. According to a particular embodiment, the target is a protein receptor. According to a particular embodiment, when *in cell*-STD NMR is performed, the target is a protein membrane receptor.

The invention also relates to a method for investigating or identifying the network of interactions, especially at the atomic level, involved in the binding between a ligand and its target.

Accordingly, the network of interactions can be investigated through several types of parameters, for example: the type of interaction involved in the ligand-target binding, the identification of the functional groups of the ligand and/or the target that are important for the binding, or the relative contribution in the binding of particular proton(s) and particular functional groups.

These parameters can be investigated from the best cluster identified and returned (provided) by the method disclosed herein, which encompasses at least one computer-generated structural model that can be considered as matching experimentally obtained structural assessment of the studied ligand-target interaction.

Accordingly, a method for investigating or identifying the network of interactions, especially at the atomic level, involved in the binding between a ligand and its target can comprise (the steps of):

a. Performing a method according to any one of the embodiments disclosed herein, providing (returning) a collection of computer-generated theoretical STD profiles, and

b. Starting from the provided (returned) collection of computer-generated theoretical STD profiles, retrieving the corresponding computer-docked ligand-target complexes, and

c. Identifying, optionally calculating, for each or most of the retrieved computer-docked ligand-target complexes the type of interactions involved in the ligand-target binding, and/or

d. Identifying the functional groups of the ligand and/or target that contribute to ligand-target binding, and optionally identifying the amino-acid residues of the target that contribute to the binding with the ligand.

The types of interactions that can be involved in the ligand-target binding may include: hydrogen bonds, electrostatic interactions, hydrophobic interactions, all of which can be easily computed using standard structure visualization and analysis program such as the commonly known UCSF Chimera program (https://www.cgl.ucsf.edu/chimera/). UCSF Chimera is a program for interactive visualization and analysis of molecular structures and related data, including density maps, supramolecular assemblies, sequence alignments, docking results, trajectories, and conformational ensembles.

Identification of functional groups and/or involved amino-acid residues can be performed visually, on a displayed 3D structure of at least one considered ligand-target model. "Contribution to binding" will be appreciated by the skilled person considering the type of interaction involved and/or the localization of the considered functional groups and/or amino-acid residues within a ligand-target complex. Integration of all available data provides information regarding important or essential functional groups and/or amino-acid residues. For example, as reported in the experiments disclosed herein, the invention enabled concluding about the importance of the T313 amino-acid residue in the IPK317-QcrB interaction.

According to another aspect, the method of the invention can, upon extraction from the collection of provided (returned) theoretical STD profiles, of the STD ratios for each proton involved in the ligand-target interaction, especially involved proton(s) as identified through STD NMR experiments, enable to determine the relative contribution of each proton to the interaction between the studied ligand and target on the basis of the STD ratio values obtained via the provided (returned) theoretical STD profiles, in particular by comparison with experimental STD ratio values. For example, as shown in Figure 4B, it is possible to represent an average of the STD ratios obtained from the best cluster provided (returned) by the method disclosed herein, and compare them to the experimentally obtained STD ratios.

The method of the invention also enables to assess whether a returned (provided) theoretical STD profile, or a cluster thereof, match experimentally obtained structural assessment of studied ligand-target interaction. As described herein, X-values can quantify the quality of the match between theoretical STD profiles and experimental STD profiles, and therefore can quantify the quality of the match between computer-generated theoretical models and a model defined on the basis of an experimental assessment thereof.

The invention also relates to a computer program product comprising software code adapted to cause a computer to perform at least steps c. to e. described herein, in particular when provided with experimental and theoretical STD profiles and Cartesian coordinates, more especially when also provided with parameters $d_i$ and $\sigma_i^2$, and optionally also adapted to cause a computer to perform all steps of a method described herein that use computer means and/or algorithms, according to any embodiment described herein.

According to a particular embodiment, a computer program product can take into account any one or all of the features described herein with respect to SOM construction, data-driven clustering and optionally conclusion step(s). Accordingly, according to particular embodiments, the computer program product comprises software code adapted to cause a computer to perform any one of the steps disclosed herein, according to all combinations thereof.

According to a particular embodiment, a computer program product further comprises software code adapted to cause a computer to perform steps required for investigating or identifying the network of interactions, especially at the atomic level, involved in the binding between a ligand and its target, as described herein.

The invention also relates to a computer readable medium having stored thereon a computer program according to any one of the embodiment(s) described herein.

Said computer readable medium can be a computer readable storage medium with a computer program stored thereon.

Accordingly, by "computer readable medium" or "computer readable storage medium", it is meant a medium capable of storing data in a format readable by a computer. According to particular embodiments, said medium can encompass primary storage, such as a memory, a RAM or a ROM, secondary storage (external memory or auxiliary storage), such as hard disk drives, CD or DVD, a flash memory (e.g. USB flash drives or keys), floppy disks, or virtual memory, or tertiary storage, such as a medium requiring the action of mounting (inserting) a removable mass storage medium(a) into a storage device according to the computer's demand.

According to a particular embodiment, a computer readable medium" or "computer readable storage medium" more particularly relates to a tangible medium, such as a computer readable medium (for example, diskette, CD-ROM, ROM, or fixed disk) or a medium for transmission to a computer system via a modem or other interface device using communications lines (for example, optical or analog communication lines) or wireless techniques (for example, microwave, infrared or other transmission techniques).

According to a particular embodiment, the invention relates to a non-transitory computer-readable storage medium with a computer program stored thereon, wherein the computer program is arranged, when executed by a microprocessor, to cause the microprocessor to perform the steps identified above and herein, according to all disclosed embodiments.

The invention also relates to a computer-displayed interface, in particular a Graphical User Interface (GUI), especially representing either or both between a U-matrix derivable from a SOM map and a similar representation such as a 2D SOM grid displaying any one of the parameters disclosed herein, especially $X_j$ value(s) as discussed and disclosed herein, or representing $X_k$-value(s) as discussed and disclosed herein.

Of note, such a U-matrix can be displayed as soon as the learning step of the SOM has been completed. Similarly, a 2D SOM grid displaying relevant parameters such as the $X_j$ values discussed and disclosed herein can be set as soon as said relevant parameters have been computed.

According to a particular embodiment, it will be understood that data relevant in the context of a computer-displayed interface, especially an interface enabling interaction with the user of the method, is especially data suitable for enabling a conclusion according to step e. described herein. As detailed herein, the skilled person can readily adapt the required visualization features to meet the user's needs. According to a particular example, the visualized data can be data further projected on a particular type of graphical representation as a substructure. For example, the display of X-type values, especially $X_j$-values, on a 2D SOM map can follow a frame obtained by displaying a U-matrix, i.e., X-type values, especially $X_j$-values, can be further projected on a 2D SOM map, the basis of which is a U-matrix. According to another embodiment or alternatively, several graphical representations can be provided side-by-side, according to all possible combinations. According to a particular embodiment, data visualization, especially for representation and/or interaction purposes, encompass $X_k$-value(s) display, alone or in combination with another type of data representation or output.

According to a particular embodiment, a Graphical User Interface of the invention is used for identifying or to identify a collection of docking solutions by applying a threshold value as disclosed in step e. described herein, and comprises means for carrying (component(s) arranged to carry) out said step e. accordingly, so as to interactively determine the threshold to apply, and means for displaying, upon application of said threshold, the resulting collection of docking solutions and any parameter associated to said collection, as in particular stored in the nodes of the SOM. According to a particular embodiment, the Graphical User Interface comprises means to select a threshold value as discussed herein, and display a collection of docking solutions defined through application of said threshold value.

According to a particular embodiment, the Graphical User Interface and/or method described herein comprise(s) display and/or interactive components arranged to carry out step e. described herein, e.g., components to interactively determine the threshold to apply, and/or components to display, upon application of said threshold, the resulting collection of docking solutions and any parameter associated to said collection, as in particular stored in the nodes of the SOM.

According to a particular embodiment, a Graphical User Interface of the invention further comprises means for displaying (component(s) arranged to display) parameters associated with (and/or mapped in) the nodes of the SOM during implementation of the method described herein, such as X-values or $X_j$ values or $X_k$-values or $X_k$-profile curves according to any embodiment described herein, or Buriedness values (B parameter), or the energy of the docked poses according to the definitions available in the art, or number of H-bonds, or distances between atoms within the docked poses, or local similarity values...

According to particular embodiments, the computer program product comprising software code or computer readable medium or computer readable storage medium with a computer program stored thereon referred to above, includes code and/or means for displaying a Graphical User Interface as described herein. According to particular embodiments, said computer program product comprising software code or computer readable medium or computer readable storage medium with a computer program stored thereon referred to above is further arranged, when executed by a microprocessor, to cause the microprocessor to display a Graphical User Interface as described herein, and provides

to the user means for interacting with said Graphical User Interface, especially for carrying out the steps descried herein involving such an interaction with the Graphical User Interface.

The skilled person can readily determine which component(s) can be arranged to act as means for as described herein, according to the practice in the art.

In an attempt to help the reader of the present application, the description has been separated in various paragraphs or sections and/or in various embodiments. These separations should not be considered as disconnecting the substance of a paragraph or section and/or of an embodiment from the substance of another paragraph or section and/or of another embodiment. To the contrary, the present application encompasses all the combinations of the various sections, paragraphs and sentences that can be contemplated. The present application encompasses all the combinations of the various embodiments that are herein described.

Other examples and features of the invention will be apparent when reading the examples and the figures, which illustrate the experiments conducted by the inventors, in complement to the features and definitions given in the present description.

LEGEND OF THE FIGURES

**Figure 1.** Workflow of the data driven clustering approach. A: The gray shading highlights the different levels of neighboring grid cells. The numbers order the grid cells in the data space (the nearest neighbor in the data space from the eight neighbors in the grid space). B: Each grid cell is representative of the theoretical STD profiles provided to the method (the data space). C: Best cluster, minimizing the $X_k$-value. (D): Average STD profiles of grid cells 0, 1, ..., 7 and corresponding $X_k$-value to experimental data.

**Figure 2.** NMR spectra of various samples. 1H NMR off-resonance spectrum of IPK317 alone (a) or in the presence of M. smegmatis qcrCABmt-WT cells (b); STD spectrum of IPK317 in the presence of M. smegmatis qcrCABmt-WT cells (c) or alone (d); STD spectrum of M. smegmatis qcrCABmt-WT cells (e); 1H NMR off-resonance spectrum of IPK317 in the presence of M. smegmatis qcrCABmt-T313A cells (f); STD spectrum of IPK317 in the presence of M. smegmatis qcrCABmt-T313A cells (g). All spectra are recorded in deuterated PBS, 0.05% Tween. The STD spectra of (IPK317)/ (cells) at the ratio of 450 are shown. Assigned protons of IPK317 are indicated in Table B.

**Figure 3.** U-matrix (a) and projections of the $X_j$-value onto the SOM maps resulting from the 50000 hit-target complex models (b). The cluster minimizing the $X_k$-value is circled in white on map (a). Representative structures extracted from cluster I, II and III are shown in (c).

**Figure 4.** (a): $X_k$-profile along the clustering process. (b): Relative STD ratio of the best cluster compared with the experimental data $X_k$=0.17. (c): Structure of IPK317, with corresponding proton numbering for signal attribution. (d): Representative hit-target complex model of the cluster minimizing the $X_k$-value.

**Figure 5.** Workflow of the approach combining *in cell* NMR and docking for rational drug design. Legend : 1 : *in cell* NMR experiments with the hit and the cells expressing the target; 1.1.1 : No STD signal ; 1.1.2 : No binding ; 1.2.1 : STD signal ; 1.2.2 : STD ratio measurement for each 1H of the hit; 2 : Hit-target complex model in silico docking ; 2.1 : SOM mapping ; 2.2 : Calculation of theoretical STD ratio for 1 H of the hit in each docking pose ; 3 : Data driven clustering of the SOM map ; 4 : Visualizing the docking poses ensemble best matching the experimental data ; 5 : Identification of the atomic network of interaction between the hit and its target.

## EXAMPLES

## A. MATERIALS AND METHODS

### Bacterial strains and plasmids used for target expression

*M. smegmatis* ΔqcrCAB[9] was kindly provided by Valerie Mizrahi and Bavesh Kana. The strain was transformed with a replicative plasmid (pMV262) harboring i) the full qcrCAB operon from *M. tuberculosis,* to yield the *M. smegmatis* qcrCABmt-WT strain, or ii) the full qcrCAB operon from *M. tuberculosis* having the mutation T313A in the QcrB subunit, to yield the *M. smegmatis* qcrCABmt-T313A strain.

**NMR sample preparation**

A stock solution of IPK317 (kind gift from the Chemical Laboratory of Institut Pasteur of Korea) at concentration of 3 mM was prepared by dissolving it in deuterated DMSO (DMSO-d6, 99.96% $^2$H atoms, *Euriso-top,* France). An aliquot of this solution was added to a 4 mm Shigemi NMR tube (*Shigemi Inc.,* USA) containing either the buffer alone or with the cell suspension. The final concentration of IPK317 in the NMR sample was 76 μM and the amount of DMSO-d6 content at 2.5 % (v/v). *M. smegmatis* ΔqcrCAB, *M. smegmatis* qcrCABmt-WT and *M. smegmatis* qcrCABmt-T313A cells were grown at 37°C in LB medium supplemented with 0,05% Tween 20. The bacterial cell suspensions were prepared just before use. Bacterial cells from overnight culture (Optical density at 600nm ($OD_{600}$) of about 1) were centrifuged. The supernatant was discarded, and the pellet was resuspended and washed with 250 μL of deuterated phosphate-buffer saline (PBS). This step was repeated three times. After the last centrifugation, the pellet was resuspended with deuterated phosphate-buffer saline (PBS), 0.05 % Tween (*Sigma-Aldrich*). Cell suspensions were at final $OD_{600}$ of 12 in the NMR tube and were then analyzed either alone, or in the presence of IPK317. The concentration of target in these cell suspensions was estimated at 0.16 μM, assuming that the average number of receptors per cell is 1000 and that 1 $OD_{600}$ =5 X $10^8$ CFU/ml (Colony Forming Unit/ml). Different ratios of the concentration of hit/target ranging from 150 to 1350 were tested.

**NMR spectroscopy**

NMR spectra were collected at 30°C on a Varian NMR System spectrometer operating at a proton frequency of 600 MHz and equipped with a cryogenically-cooled triple resonance $^1H\{^{13}C/^{15}N\}$ PFG probe (*Agilent Technologies,* USA).

IPK317 resonances were assigned with standard 1D $^1$H, 1D$^{19}$F and 2D $^1$H-$^1$H TOCSY experiments (mixing time of 65 ms). *In cell*-STD spectra were acquired by using the standard pulse sequence implemented from the VnmrJ Biopack (*Agilent Technologies,* USA) with on- and off-resonance saturation frequencies of 0.0 and 35.0 ppm, respectively. The on- and off-resonance spectrum were obtained by a train of 40 selective E-BURP-2 pulses of 50 ms each[10], separated by a 1 ms delay, with a total saturation time of 2 s. Water suppression was achieved by a double PFG spin-echo[11]. A T1rho filter of 10 ms was used to remove spectral components arising from the target. The off-resonance spectrum was used as reference spectrum. Control experiments with only the hit or only the bacterial cell suspension were acquired under the same conditions.

The STD ratio corresponds to the ratio of the area of each signal in the STD NMR spectrum ($A_{STD}$) to that of the same signal in the reference spectrum ($A_0$)[3] (see section 2.1.5 and scheme 3 of Reference 3). The ratio ($A_{STD}/A_0$) was normalized to a maximum value of 1, belonging to the hit atom closest to the target, receiving therefore the maximum of irradiation from the target atoms. The STD ratios used for the docking are the average of 4 different experiments with at least 2 independent batches of cells. The $^1$H chemical shifts were referenced to external 2,2-dimethyl-2-silapentane-5-sulfonate.

Therefore, for the purpose of obtaining experimental STD profiles, a off-resonance spectrum of the ligand in interaction with the receptor was compared with the STD spectrum of the same sample (nota: a 1 H 1 D NMR spectrum could conversely have been used). The peak integrals were determined for each signal of the ligand. The profile of the ligand, i.e., the experimental STD profile, was obtained by making the ratio between the peak integrals of the STD signals over the peak integrals of the off-resonance spectrum. These ratios were normalized 1 for the proton(s) with the highest STD ratio..

**3D model of *M. tuberculosis* QcrB**

A 3D model of the target or *M. tuberculosis* QcrB ($QcrB_{Mt}$) was obtained by comparative modeling with the Phyre2 pipeline[12], using the default parameters. The model with the highest confidence and the best alignment coverage was selected. The template for this model was the crystal structure of mutant *Rhodobacter sphaeroides* bc1 with stigmatellin and antimycin inhibitors (PDB code: 2QJK). The transmembrane helices were predicted with Memsat SVM 3[14].

The $QcrB_{Mt}$ model was then refined in the "Relax" application of Rosetta[15]. The goal of this step is to energetically minimize the structure and to explore the local conformational space of the protein. This method has been shown to significantly lower the overall energy of the model and to be essential for achieving atomic detail accuracy. To deal with the protein flexibility during the docking procedure, 1000 conformations were generated with the Relax protocol from Rosetta[15]. The number of conformations was set to 1000 to obtain a reasonable sampling with a limited time of computation.

## Docking procedure

The IPK317 molecule was docked onto the 1000 conformations of $QcrB_{Mt}$ generated by Rosetta Relax. To sample the conformational space of the bound state of the hit compound, 50 poses were generated for each conformations of $QcrB_{Mt}$, leading to a total of 50,000 models of the complex. The docking calculation was performed with Dock 6.5 [16].

The Dock program uses spheres to obtain a representation of the empty space defining the cavity. The spheres of the 1000 conformations generated were computed with sphgen_cpp. The minimum sphere radius was set to 1.4 Å and the maximum radius to 4.0 Å (default parameters of sphgen_cpp). The spheres defining the pocket for the docking were defined from the crystallographic structure of the template (PDB code: 2QJK) bound to the ligand stigmatellin[13] structurally aligned onto the 3D model. All spheres within 10.0 Å from every atom of the crystal structure of the ligand were selected. To evaluate rapidly the interaction energy between the docked molecule and the protein, the Dock program uses a grid representation of the inter-atomic interactions. The 1000 grids (one grid/ conformation) were generated with the grid utility from the Dock 6.5 suite with standard parameters.

The grid spacing was set to 0.3 Å, without energy cut-off distance, and attractive exponent and repulsive exponent for Van der Waals were set to 6 and 12, respectively. The dielectric factor was set to 4, with bump filter active and the bump overlap set to 0.75. The Dock program cuts the docked molecule into rigid fragments (anchor segments) and docked them individually before merging them to obtain the final docking pose. A pruning procedure was used to keep only the most favorable orientations of the anchors. This iterative reconstruction of the ligand conformation in the docking site is called anchor and grow procedure. The default parameters were used for the docking step. More particularly, the docking was performed with a maximum number of orientations for the ligand set to 1,000 and a minimum anchor size set to 5 heavy atoms for an anchor segment. Clustering was enabled during the pruning algorithm, with a maximum number of anchor orientations carried forward in the anchor and grow search set to 100. The pruning value cut-off for anchor orientations promoted to the conformational search was set to 100. The maximum score for conformation after minimization was set to 25.0. The van der Waals scale of the grid score was set to 0.5. This setup increases the conformational flexibility of the bound form of the ligand by decreasing the penalty due to steric clash with the protein. The Hawkins GB/SA score implementation of the Molecular Mechanics Generalized Born Surface Area (MM-GBSA) method with default parameters was used as a secondary score. More particularly, the solvent dielectric was set to 78.5, the salt concentration to 0.15 M and the attractive exponent and repulsive exponent were set to 6 and 12 respectively.

## Computation of theoretical Saturation Transfer Difference (STD) relative intensities of the docking poses

Theoretical STD ratios were computed for each observable proton of the hit so as to mimic the corresponding experimental STD ratios measured as described above in section "NMR spectroscopy". These STD ratios were normalized to a maximum value of 1.0 for the most buried proton of the hit in the binding pocket. For each 50,000 docking poses, the buriedness of the protons were computed as follows:

- All protons of the target around the protons of the hit were selected by a KDTree neighbor search.
- For each proton of the hit, the buriedness was defined as the number of protons of the target in a sphere of 6.0 Å around the hit proton, divided by the total number of protons of the target closer than 6.0 Å to any proton of the hit.
- When several hit protons were equivalent (presenting the same chemical shift), the mean buriedness value was computed. The theoretical STD profiles of each of the 50 000 hit-target complexes were compared with the experimental ones by computing X-values (where $x_i$ is the computed values and $d_i$ the experimental data with corresponding variance $\sigma_i^2$).

$$\chi = \sqrt{\sum_{i=1}^{n} \frac{(x_i - d_i)^2}{\sigma_i^2}}$$

## Data driven clustering of docking poses using STD data

The Self-Organizing Map (SOM) has been shown to be very efficient for the clustering of docking poses[7,17] and molecular conformations[18]. Complexes of $QcrB_{Mt}$ with IPK317 were clustered with SOM. Cartesian coordinates of the hit were used as input for the SOM after prior structural alignment of the target. Default parameters were used to train the SOM:

- A SOM map of size of 50 X 50;

- 2 training phases with 50 000 iterations for the first one and 100 000 for the second one;
- An exponentially decreasing learning rate of 0.5 to 0.25 for the first phase and from 0.25 to 0 for the second one;
- An exponentially decreasing Gaussian radius of 6.25 to 3.125 for the first phase and from 3.125 to 1.0 for the second one.

These parameters have previously been shown to be efficient for the clustering of structural data[18].

The SOM can be seen as a projection of the data space (the space containing the STD profiles) onto a grid space. In this grid space, one can easily define the eight neighbors from any grid point. Among these eight neighbors, one can define the closest one in the data space, thus ordering the grid cells from any chosen starting point ending up with a list of ordered grid cells from the closest one to the furthest one (Figure 1).

Flooding: In order to analyse the resulting SOM, the map was depicted by building a minimum spanning tree. For each cell, the eight neighbours were defined by the Cartesian grid of the SOM defining a regular graph of degree 8. From this tree, a minimum spanning tree was computed with the Kruskal algorithm. From this object, using the Dijkstra algorithm, one can compute the shortest distance of each cell from a given one defined as the starting cell. From this data object, the nearest neighbours of an arbitrary structure can quickly be picked up from the ensemble of structures (the Kruskal algorithm therefore returns a graph which contains all possible solutions, and the Dijkstra algorithm enables the identification of the best local solution from a starting cell). From any neuron, one can define an ordered list of neurons, starting from the nearest to the furthest one. This list of neurons is called flooding by analogy to the path of water during flooding. For each flooding step, i.e., for each element of this list, an average theoretical STD profile can be computed from all the elements preceding and the corresponding $X_k$-value calculated. This profile displays a global minimum that can be used as a threshold to define a cluster. In this way, a cluster can be defined for each grid cell of the map. However, a lot of partially or totally overlapping clusters are built. For overlapping clusters, only those with lowest $X_k$-values are kept.

## B. RESULTS

### Interaction of IPK317 with entire cells expressing QcrCABMt

To avoid handling of pathogenic bacteria and their insertion into the NMR spectrometer, QcrCAB ($QcrCAB_{Mt}$) was reconstituted in *M. smegmatis,* commonly used as non pathogenic model-system instead of *M. tuberculosis*. A mixture of IPK317 at 76 μM and freshly prepared M. smegmatis qcrCABmt-WT cell suspensions containing approximately 0.16 μM of target were used for the STD experiments. The mixture remained sufficiently homogenous during six hours to obtain an interpretable and reproducible STD signal. All experimental parameters were therefore optimized in order to limit the duration of experiments to 4 hours.

In the 1D $^1H$ spectrum of the mixture of IPK317 with cells, the signals from the aromatic protons of the drug can easily be identified (Figure 2a). They appear at 6.5 to 8.5 ppm. The signals from aliphatic protons of IPK317 can also be recognized, though slightly overlapping with the signals belonging to various components of the mixture (cells and buffer) (Figure 2a). All signals corresponding to IPK317 protons were assigned from a set of $^1H$ and $^{19}F$ NMR experiments, apart from proton 6 which is exchanged and not observed and the proton 7,7' overlapping with aliphatic protons from the buffer (Chemical structure (A) herein and Figure 2).

The STD spectrum of the mixture shows different peaks corresponding to the protons of the hit compound (Figure 2c). The presence of IPK317 signals indicates that their corresponding atoms are in contact with the target protons saturated with the on-resonance irradiation at 0 ppm. Control STD experiments with each of the components alone, that is, the hit in the buffer or the cell suspension, do not display any peaks corresponding to IPK317 (Figure 2d and e).

The QcrB T313A mutation was shown to confer resistance to IP drugs. The STD experiment with IPK317 in the presence of cells expressing QcrCABMt_T313A do not show any signal corresponding to IPK317 (Figure 2g versus 2c). The absence of STD signal signifies that IPK317 does not interact with $QcrCAB_{Mt_T313A}$. Therefore the replacement of T313 of QcrB by an alanine abolishes the interaction of IPK317-QcrB.

### Model building of the hit-target complex

The 1000 target conformers that we generated with the Rosetta Relax algorithm provide a reliable representation of the local target flexibility. For each conformer, we retained 50 models of hit-target complex produced by the docking procedure, which makes a total of 50000 complex models. The SOM algorithm is ideally suited to extract meaningful information from this large amount of structural data. First of all, it gives a way to project the high dimensional data to a 2D space or a grid that is easy to visualize. This feature is of great help to explore the data interactively. This exploration is facilitated by a Graphical User Interface (GUI) developed by the inventors, which allows the user to directly pick a

conformation from the SOM space and look at the corresponding 3D model of hit-target complex. In addition, the SOM gives a way to project any other data related to the structure of the complex (energy, number of h-bonds, X-type value, ...) onto the 2D SOM grid, making it possible to easily annotate the realized mapping and to highlight interesting parts of the conformational landscape.

In order to visualize the SOM, it is usual to define the U-matrix (Figure 3a). This matrix is built by computing the average distance between each grid cell and its eight direct neighbors. The resulting matrix depicts the local similarity of the projection and gives access to the topology of the sampled conformational space. The basins, in black in the matrix, can be interpreted as clusters of very similar structures. In Figure 3b, the $X_j$-values corresponding to the 50000 generated hit-target complex models are projected onto the SOM. This visualization can be used to identify clusters of models that fit the experimental data well. Applying a threshold of 20 A on the U-matrix delineates 2 main structural clusters, depicted by I and III on Figure 3a. The threshold has been defined as $\mu$+2$\sigma$, with $\mu$, the average value of the U-matrix and $\sigma$, its standard deviation. These two clusters are not contiguous and a transition cluster (II) lies in between the two. The corresponding models of the hit-target complex are characterized by different docking positions relative to a transient cavity only present in the transition clusters (II) (Figure 3c).

### Data driven clustering

Each grid cell of the SOM was sorted from the closest one to the furthest one in the data space as described in Materials and Methods. The evolution of the $X_k$-value along this process results in the $X_k$-profile curve presented in Figure 4a. From this plot, an obvious global minimum can be found, defining the cluster limits for the chosen grid cell.

The global cluster minimizing the $X_k$-value is circled in white in Figure 3a. The corresponding $X_k$-value is 0.17. This cluster is in the region where the transient pocket is opened. The corresponding theoretical STD relative intensities are depicted in Figure 4b and c, and are strongly correlated with experimental data. The representative 3D structure of the hit-target complex model shows that the hit lies deeply in the transient pocket detected (cluster II in Figure 3a). Two main hydrogen bonds with T313 and E314 stabilize the hit along with H195 and R318, albeit less frequently.

The data-driven clustering approach allowed us to identify a transient pocket on the target that binds the hit specifically. The main interactions involved in this binding were identified. The resulting theoretical STD profile correlates strongly with experimental data. The residue T313 identified in the interaction network and stabilizing the hit in the complex model is a key residue, since its mutation to alanine confers the resistance. This result is a double validation of our model as it validates the QcrB protein as the target (specificity) as well as its identified binding mode.

### C. DISCUSSION

The approach described herein, combining *in cell* NMR, docking and efficient clustering driven by cautiously defined and new parameters taking into account the experimental reality, makes it possible to set up a pipeline for obtaining crucial information on hit-target interactions at atomic resolution. It is the only approach that provides such information in the context of living cells, in solution, and that takes into account the dynamics of the molecular associations. The different steps of this approach are summarized in the diagram of Figure 5.

The *in cell*-STD experiment can be applied when the target is a receptor or a surface protein. The experimental feasibility can be quickly tested with only one step of bacterial culture. The only experimental limitation, considering *in cell*-STD experiments, remains the solubility of the drug, which should be at least in the $\mu$M range in a buffer compatible with the viability of cells. The overexpression of the target, though non mandatory, makes it possible to use reasonable amounts of bacteria cells, which can be maintained in a homogenous liquid state and a small volume of a NMR tube (from 250 to 700 $\mu$L) during the time of the experiment. The control experiment can be easily implemented by making use of the cells without target overexpression or expression.

When using the *in cell* strategy, the experimental conditions are as close as possible to physiological and cellular conditions. In this way, it is possible to rapidly test the binding of a set of drugs or hits to their target in the context of whole living cells, without any particular treatment. Moreover, this protocol represents a significant progress in hit-target interaction studies, since the time- and money- consuming steps of extraction, purification and stabilization of the target are avoided.

The docking of a large number of hit-target complexes and their clustering and comparison to NMR data leads to an ensemble of conformation. This approach provides a realistic view of hit-target complex by taking into account the dynamics of the molecular association. Both partners are flexible during the docking step. Such a fully flexible docking procedure is not often used as it significantly increases the number of conformations to be generated to explore as much as possible the degrees of freedom. Without an efficient analysis method, based on a machine learning procedure, and structural experimental data, the high number of structural models generated cannot be handled and sorted out. If the 3D structure of the target, required at this stage, is unknown, it can be modeled based on known structures of its homologues.

The results of the IPK317-QcrB interaction study validate the combined approach presented here. Besides NMR experimental data, no other information was used for docking and clustering. The generated ensemble of hit-target complexes allowed us to identify the network of interactions that are crucial for binding. The fact that in the ensemble of conformation the hit is in contact with the residue T313, the site of the spontaneous mutation that confers the resistance[8], represents an independent validation of the method of the invention.

The combined approach devised in this study will be highly advantageous in many modern drug development programs. The atomic information on hit-target interaction is crucial for the step of hit-to-lead optimization. More generally, this original approach can be very valuable for any small molecule-receptor complex study.

References

(1) Homans, S. W. Angew Chem Int Ed Engl 2004, 43, 290.
(2) Pellecchia, M.; Bertini, I.; Cowburn, D.; Dalvit, C.; Giralt, E.; Jahnke, W.; James, T. L.; Homans, S. W.; Kessler, H.; Luchinat, C.; Meyer, B.; Oschkinat, H.; Peng, J.; Schwalbe, H.; Siegal, G. Nat Rev Drug Discov 2008, 7, 738.
(3) Meyer, B.; Peters, T. Angew Chem Int Ed Engl 2 003, 42, 864.
(4) Claasen, B.; Axmann, M.; Meinecke, R.; Meyer, B. J Am Chem Soc 2005, 127, 916.
(5) Mari, S.; Serrano-Gomez, D.; Canada, F. J.; Corbi, A. L.; Jimenez-Barbero, J. Angew Chem Int Ed Engl 2004, 44, 296.
(6) Assadi-Porter, F.M.; Tonelli, M.; Maillet, E.; et al. J Am Chem Soc 2008, 130, 7212.
(7) Bouvier, G.; Evrard-Todeschi, N.; Girault, J. P.; Bertho, G. Bioinformatics 2010, 26, 53.
(8) Pethe, K.; Bifani, P.; Jang, J.; Kang, S.; Park, S.; Ahn, S.; Jiricek, J.; Jung, J.; Jeon, H. K.; Cechetto, J.; Christophe, T.; Lee, H.; Kempf, M.; Jackson, M.; Lenaerts, A. J.; Pham, H.; Jones, V.; Seo, M. J.; Kim, Y. M.; Seo, M.; Seo, J. J.; Park, D.; Ko, Y.; Choi, I.; Kim, R.; Kim, S. Y.; Lim, S.; Yim, S. A.; Nam, J.; Kang, H.; Kwon, H.; Oh, C. T.; Cho, Y.; Jang, Y.; Kim, J.; Chua, A.; Tan, B. H.; Nanjundappa, M. B.; Rao, S. P.; Barnes, W. S.; Wintjens, R.; Walker, J. R.; Alonso, S.; Lee, S.; Kim, J.; Oh, S.; Oh, T.; Nehrbass, U.; Han, S. J.; No, Z.; Lee, J.; Brodin, P.; Cho, S. N.; Nam, K.; Kim, J. Nat Med 2013, 19, 1157.
(9) Matsoso, L. G.; Kana, B. D.; Crellin, P. K.; Lea-Smith, D. J.; Pelosi, A.; Powell, D.; Dawes, S. S.; Rubin, H.; Coppel, R. L.; Mizrahi, V. J Bacteriol 2005, 187, 6300.
(10) Geen, H.; Freeman, R. JMagn Reson 1991, 93, 93.
(11) Hwang, T. L.; Shaka, A. J. J Magn Reson Ser A 1995, 112, 275.
(12) Kelley, L. A.; Mezulis, S.; Yates, C. M.; Wass, M. N.; Sternberg, M. J. Nat Protoc 2015, 10, 845.
(13) Esser, L.; Elberry, M.; Zhou, F.; Yu, C. A.; Yu, L.; Xia, D. J Biol Chem 2008, 283, 2846.
(14) Jones, D. T. Bioinformatics 2007, 23, 538.
(15) Combs, S. A.; Deluca, S. L.; Deluca, S. H.; Lemmon, G. H.; Nannemann, D. P.; Nguyen, E. D.; Willis, J. R.; Sheehan, J. H.; Meiler, J. Nat Protoc 2013, 8, 1277.
(16) Lang, P. T.; Brozell, S. R.; Mukherjee, S.; Pettersen, E. F.; Meng, E. C.; Thomas, V.; Rizzo, R. C.; Case, D. A.; James, T. L.; Kuntz, I. D. RNA 2009, 15, 1219.
(17) Mantsyzov, A. B.; Bouvier, G.; Evrard-Todeschi, N.; Bertho, G. Adv Appl Bioinform Chem 2012, 5, 61.
(18) Bouvier, G.; Desdouits, N.; Ferber, M.; Blondel, A.; Nilges, M. Bioinformatics 2015, 31, 1490.
(19) Juha Vesanto, Intelligent Data Analysis, Volume 3, Issue 2, 1999, Pages 111-126


## Claims

1. Computer-implemented method of identifying a collection of docking solutions representing a ligand-target interaction, which matches experimentally obtained structural assessment of said ligand-target interaction, said method comprising the steps of:

a. Obtaining, from several Saturation Transfer Difference - Nuclear Magnetic Resonance (STD-NMR) experiments investigating the studied ligand-target interaction, an average experimental STD NMR profile consisting of a discrete collection of *n* STD ratios (*n* an integer), where each STD ratio is defined for each of the *n* protons of the ligand involved in the ligand-target interaction, in which for each proton *i* (*i* an integer, $1 \leq i \leq n$) an average experimental STD value $d_i$ is provided, each average experimental STD value $d_i$ being associated with a corresponding variance $\sigma_i^2$ ;

b. Obtaining, from *N* (*N* an integer) computer-docked ligand-target complexes, especially obtained by molecular

docking or molecular dynamics simulation:

- A set of N computer-generated theoretical STD profiles, each profile consisting of a discrete collection of n theoretical STD ratios, where each theoretical STD ratio is defined for each of the n protons of the ligand determined to be involved in the ligand-target interaction, and;
- A set of *N* cartesian coordinates of the ligand docked in said ligand-target complexes;

c. Constructing a self-organizing map (SOM) having P (*P* an integer, $P \leq N$) nodes, where:

- The SOM is trained with the set of *N* cartesian coordinates of the ligand of step b., and
- The SOM is mapped with the N **computer-generated theoretical STD** profiles of step b., and
- For each node P of the mapped SOM, a **mean theoretical STD profile** is calculated using the theoretical STD profiles of the preceding step allocated to said node P, and
- Optionally, calculating for each node P of the mapped SOM, a $X_j$-value according to the formula:

$$\chi_j = \sqrt{\sum_{i=1}^{n} \frac{(MeR_{theoi} - d_i)^2}{\sigma_i^2}}$$

wherein

*j* is an integer between 1 and *P*, and
*n* is the number of observed protons, and
$MeR_{theoi}$ is a mean theoretical STD ratio for each of the *n* protons of the mean **theoretical STD profile,** and
*i* ranges from 1 to *n, and*
$d_i$ is the experimental STD value defined in step a. for each *n* proton,
$\sigma_i^2$ is the variance associated with the experimental STD value $d_i$

d. Optionally, clustering the *P* **mean theoretical STD profiles** of the SOM, through:

i. Ordering the nodes of the SOM, and returning an ordered list of up to *P* nodes, the ordering of the nodes being obtained through a flooding algorithm, and
ii. For each node *k* (*k* an integer, $1 \leq k \leq P$) of the ordered list of up to *P* nodes, computing an **average theoretical STD profile** averaging the mean theoretical STD profiles of all the preceding *P* nodes, including the instant node *k,* said **average theoretical STD profile** consisting of a discrete collection of *n* average theoretical STD ratios $AvR_{theo}$, where each average theoretical STD ratio ($AvR_{theoi}$ (*i* an integer, $1 \leq i \leq n$)) is defined for each of the n protons of the ligand determined to be involved in the ligand-target interaction, and
iii. Calculating for each node *k* of the ordered list of up to *P* nodes, an $X_k$-value according to the formula (a weighted least-square method):

$$\chi_k = \sqrt{\sum_{i=1}^{n} \frac{(AvR_{theoi} - d_i)^2}{\sigma_i^2}}$$

wherein

*k* is an integer between 1 and *P,* and
*n* is the number of observed protons, and
$AvR_{theoi}$ is an average theoretical STD ratio for each of the *n* protons of the average **theoretical STD profile,** and
*i* ranges from 1 to *n, and*
$d_i$ is the experimental STD value defined in step a. for each *n* proton,
$\sigma_i^2$ is the variance associated with the experimental STD value $d_i$

iv. Providing a **$X_k$-profile curve** displaying the computed $X_k$-value(s) in ordinate and the ordered list of up to *P nodes* in abscissa, and

e. Identifying a collection of docking solutions by:

- applying a **threshold value** with respect to the $X_j$-values associated with the nodes of the SOM calculated in step c., wherein the threshold value is pre-determined and/or interactively determined, especially interactively determined through a computer-displayed interface such as defined in claim 15, or through interaction with a 2D SOM grid displaying $X_j$-values, and the collection of docking solutions consists of all the computer-generated mean theoretical STD profiles of the nodes of the SOM, which are associated with a computed $X_j$-value that is equal or below said threshold, and/or
- applying a **threshold value** to the $X_k$-profile curve obtained in step d. iv., wherein the threshold value is determined as corresponding to a **local** minimum value $X_{min-loc}$ on the $X_k$-profile curve, the collection of docking solutions consisting of all the computer-generated mean theoretical STD profiles of the nodes of the SOM, which result, after implementation of step d., in an $X_k$-value on the $X_k$-profile curve that is equal or below the local minimum value $X_{min-loc}$, or
- applying a **threshold value** to the $X_k$-profile curve obtained in step d. iv., wherein the threshold value corresponds to a **global** minimum value $X_{min}$ on the $X_k$-profile curve, the collection of docking solutions consisting of all the computer-generated mean theoretical STD profiles of the nodes of the SOM, which result, after implementation of step d., in an $X_k$-value on the $X_k$-profile curve that is equal or below the global minimum value $X_{min}$,

and optionally providing the collection of identified docking solutions.

**2.** The method of claim 1, wherein an average **experimental STD NMR profile** in step a. is obtained by averaging STD ratio values individually corresponding to several **experimental STD NMR profile, wherein each experimental STD NMR profile** is represented by a collection of several (n) STD ratios, wherein each STD ratio is a ratio defined for each observable proton of the ligand involved in the ligand-target interaction, and wherein a STD ratio is defined as the ratio ($A_{STD}/A_0$) of the area of a proton signal in a STD NMR spectrum ($A_{STD}$) to that of the same signal in a reference spectrum (Ao), normalized to a maximum value set to 1 for the proton(s) with the highest STD signals.

**3.** The method of any one of claims 1 or 2, wherein a **computer-generated theoretical STD profile** in step b. is represented by a collection of ratios, each ratio value being defined for each observable proton of the ligand involved in the ligand-target interaction as a parameter B, wherein the parameter B is defined as the number of protons of the target in a sphere of diameter between 5.0 Å and 7.0 Å around the considered ligand proton divided by the total number of protons of the target encompassed within the spheres centered at each proton of the ligand, and the parameter B is normalized to a maximum value set to 1 for the most buried proton of the ligand within the ligand-target interaction.

**4.** The method according to claim 3, wherein:

- the protons of the ligand are determined by comparison with and/or according to the protons of the ligand observable by 1 H 1 D NMR and/or STD NMR experiments, and
- the protons of the target around the protons of the ligand are selected using a nearest neighbor search algorithm.

**5.** The method of any one of claims 1 to 4, wherein the computer-generated theoretical STD profiles and Cartesian coordinates in step b. are obtained:

◦ By providing or generating one or several conformations of the molecular structure of the studied target, especially between 500 and 1500 conformations, in particular from a pre-existing conformational template model of said target molecular structure or a conformational template model build by a protein conformational sampling method, especially generating an ensemble of conformations representing the studied ligand-target interaction site, and then
◦ Generating, for each conformation of the target retrieved from the above step, several poses of the studied ligand-target complex, by docking several poses, especially between 20 and 70 poses, of a conformational molecular model of the ligand onto a single target conformation, using a computer-implemented docking program, Or,
◦ By generating several poses of the studied ligand-target complex by molecular dynamics simulation,

And retrieving several, especially N, generated molecular models of the studied ligand-target complex and obtaining their corresponding **computer-generated theoretical STD profiles, and optionally retrieving the corresponding** cartesian coordinates of the ligand docked in said ligand-target complexes.

**6.** The method of any one of claims 1 to 5, wherein the SOM:

- Has a size that is defined by a *l* per *r* array of nodes, with *l* ranging from 10 to 100 and *r* ranging from 10 to 100, especially a size of 50 per 50, and/or
- Is trained with at least one training phase, in particular a first training phase with a number of iterations that is roughly equal to the number of input data used for training, and optionally with a second training phase that has a number of iterations that is two times the number of iterations used in the first training phase, and/or
- Is trained with an exponentially decreasing learning rate, and/or
- Is trained with an exponentially decreasing Gaussian radius.

**7.** The method of any one of claims 1 to 6, wherein the STD NMR experiments of step a. are *in cell*-STD NMR experiments, especially *in cell*-STD NMR experiments carried out on whole bacterial cells, in particular wherein STD NMR experiments are performed and their spectra acquired.

**8.** The method according to claim 7, wherein *in cell*-STD NMR is carried out and spectra are acquired by a standard pulse sequence, with on- and off-resonance saturation frequencies of 0.0 and 35.0 ppm, with the on-resonance spectrum obtained by a train of 40 selective E-BURP-2 pulses of 50 ms each, separated by a 1 ms delay, with a total separation time of 2 s, optionally with the application of a Th1rho filter of 10 ms, the off-resonance spectrum being used a reference spectrum.

**9.** The method of any one of claims 1 to 8, wherein the ligand is selected amongst: a peptide, an oligopeptide, a nucleic acid molecule such as DNA or a RNA molecule, a metabolite, a carbohydrate or structural variant thereof, a natural or synthetic chemical compound such as a drug.

**10.** The method of any one of claims 1 to 9, wherein the target is selected amongst: a protein, a protein receptor, a membrane-bound protein receptor, a membrane-embedded protein receptor.

**11.** Method of investigating or identifying the network of interactions, especially at the atomic level, involved in the binding between a ligand and its target, comprising the steps of:

a. Performing a method according to any one to claim 1 to 10, providing a collection of computer-generated theoretical STD profiles, and
b. From the provided collection of computer-generated theoretical STD profiles, retrieving the corresponding computer-docked ligand-target complexes, and
c. Identifying, optionally calculating, for each or a part of the retrieved computer-docked ligand-target complexes the type of interactions involved in the ligand-target binding, and/or
d. Identifying the functional groups of the ligand and/or target that contribute to ligand-target binding, and optionally identifying the amino-acid residues of the target that contribute to the binding with the ligand.

**12.** A computer program product comprising software code adapted to cause a computer to perform at least steps c. to e. of any one of claims 1 to 10, in particular when provided with experimental and theoretical STD profiles and Cartesian coordinates, especially when further provided with parameters $d_i$ and $\sigma_i^2$, and optionally also adapted to cause a computer to perform all steps of claim 1.

**13.** The computer readable product according to claim 12, which further comprises software code adapted to cause a computer to perform any one or several or all of steps b., c. and/or d. of claim 11.

**14.** A computer readable medium having stored thereon the computer program of claim 12 or 13.

**15.** A computer-displayed interface, in particular a Graphical User Interface (GUI), especially representing either or both between a U-matrix derivable from a SOM map and a 2D SOM grid displaying $X_j$-values, or representing $X_k$-values as defined in claim 1, the SOM being as defined in any one of claim 1 or 6, to identify a collection of docking solutions by applying a threshold value according to step e. of claim 1, comprising means for carrying out step e. of claim 1, so as to interactively determine the threshold to apply, and means for displaying, upon application of said threshold,

a resulting collection of docking solutions.

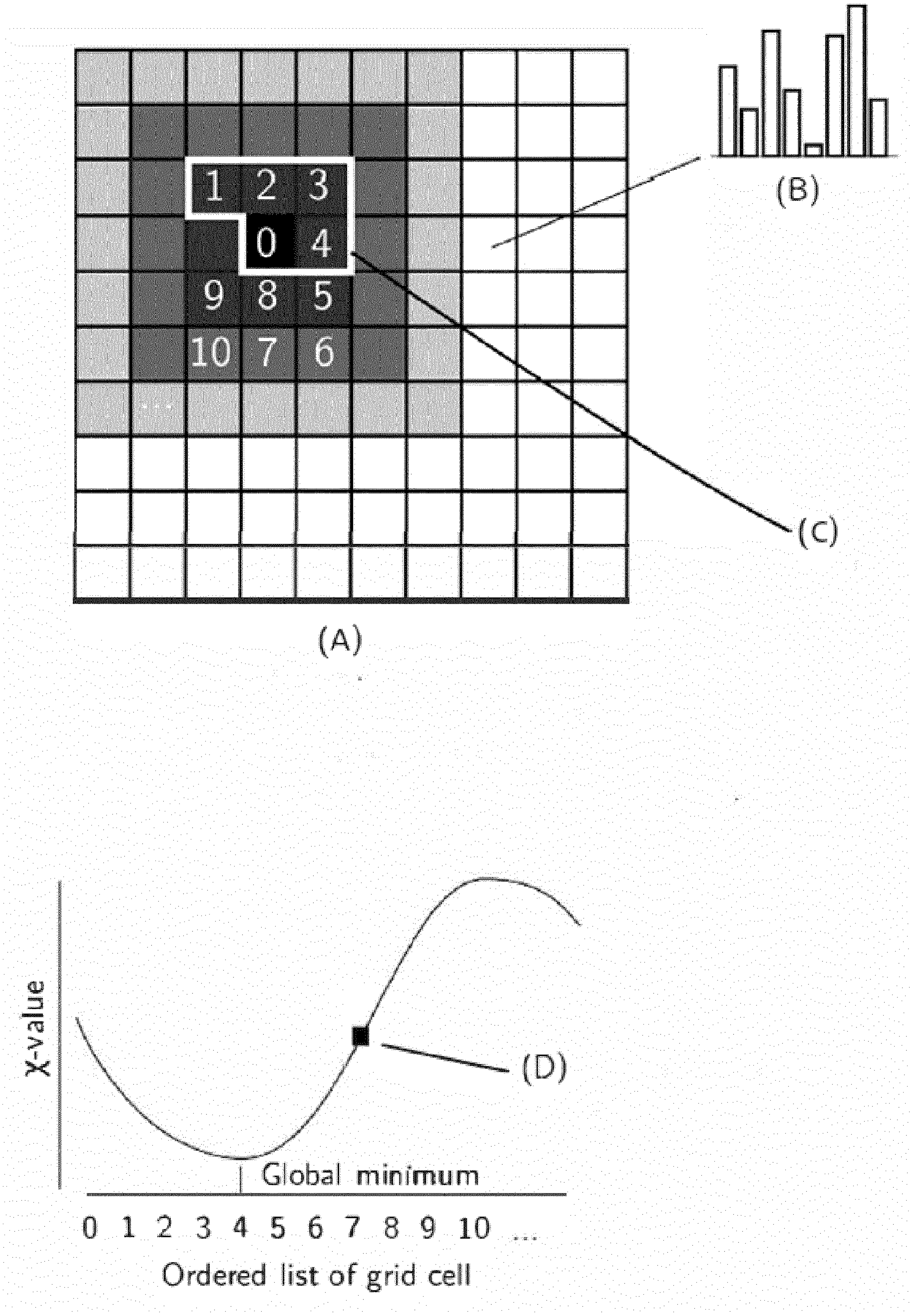
1
2
3
0
4
9
8
5
10
7
6
...
(A)
(B)
(C)
(D)
χ-value
Global minimum
0 1 2 3 4 5 6 7 8 9 10 ...
Ordered list of grid cell

FIG. 1

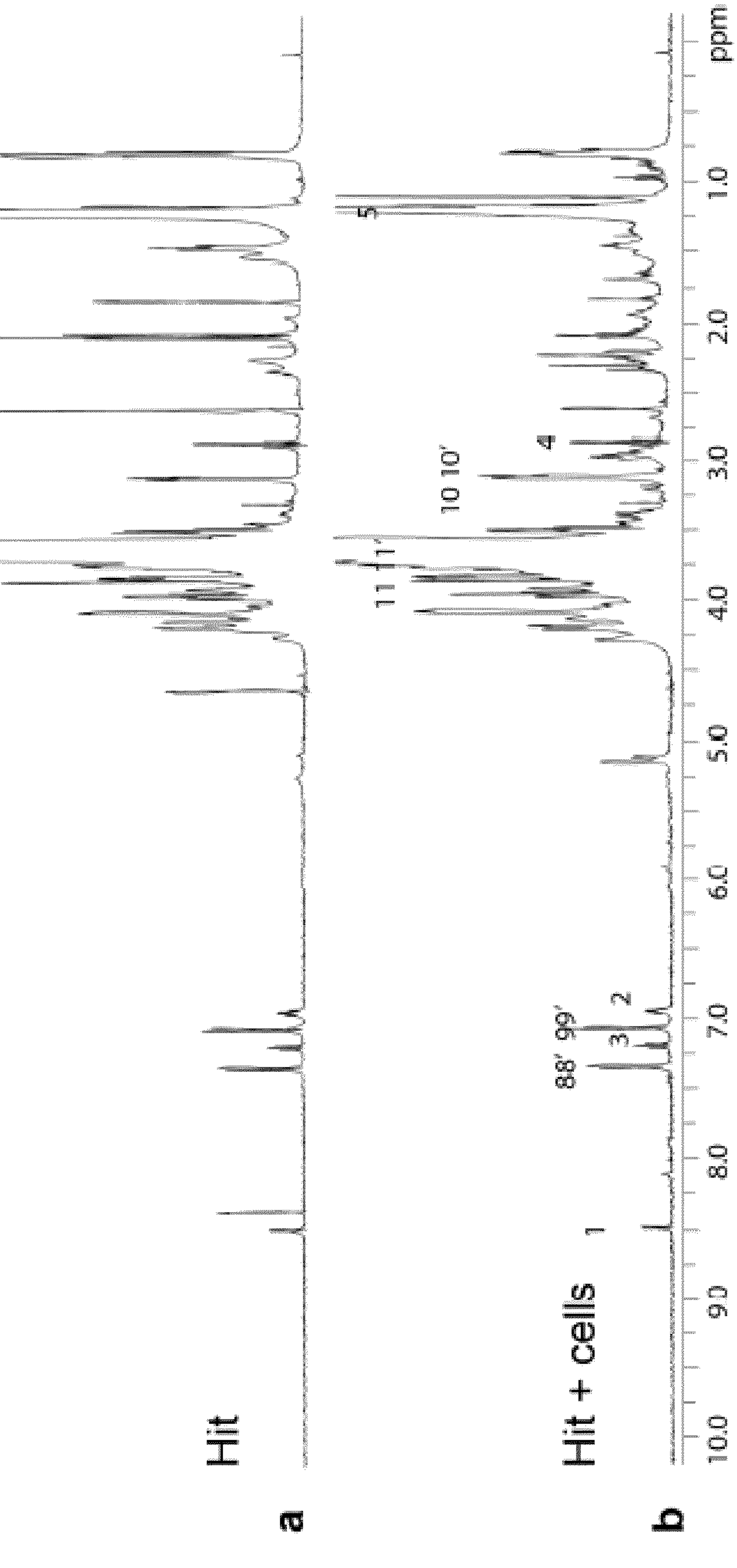
a
Hit
b
Hit + cells
ppm
10.0
9.0
8.0
7.0
6.0
5.0
4.0
3.0
2.0
1.0
1
88' 99'
3
2
11 11'
10 10'
4
5

FIG. 2A, 2B

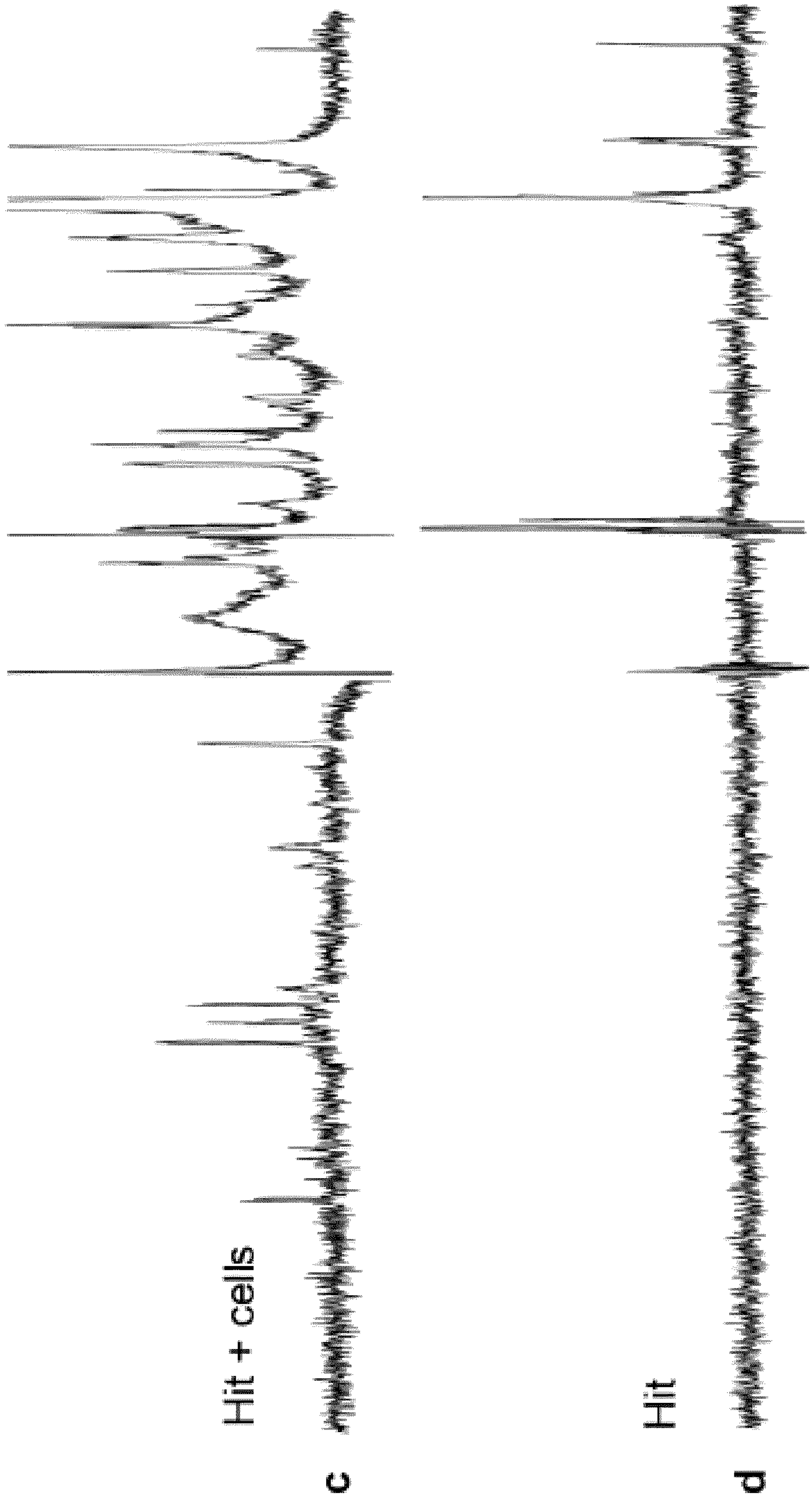


FIG. 2C, 2D

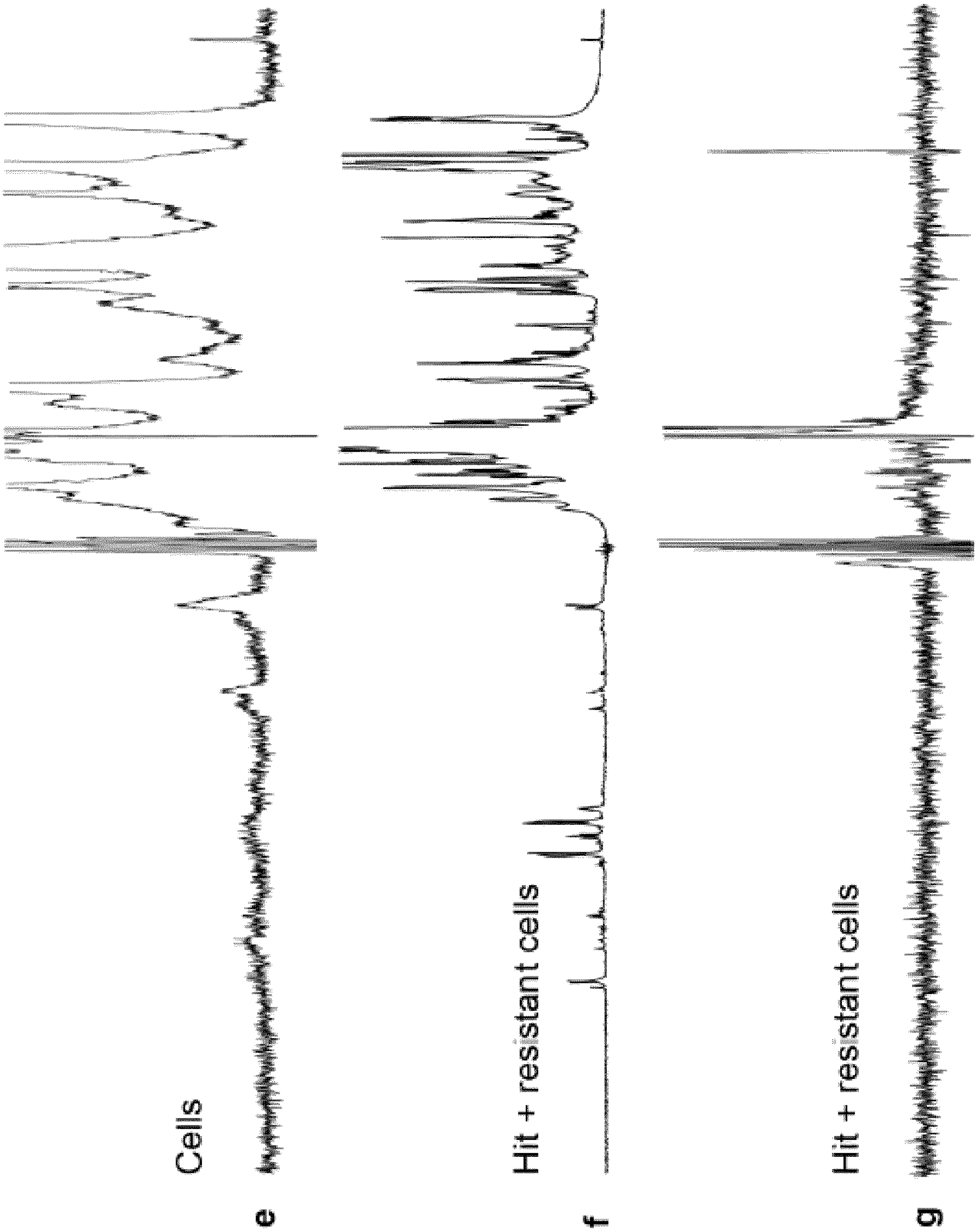


FIG. 2E, 2F, 2G

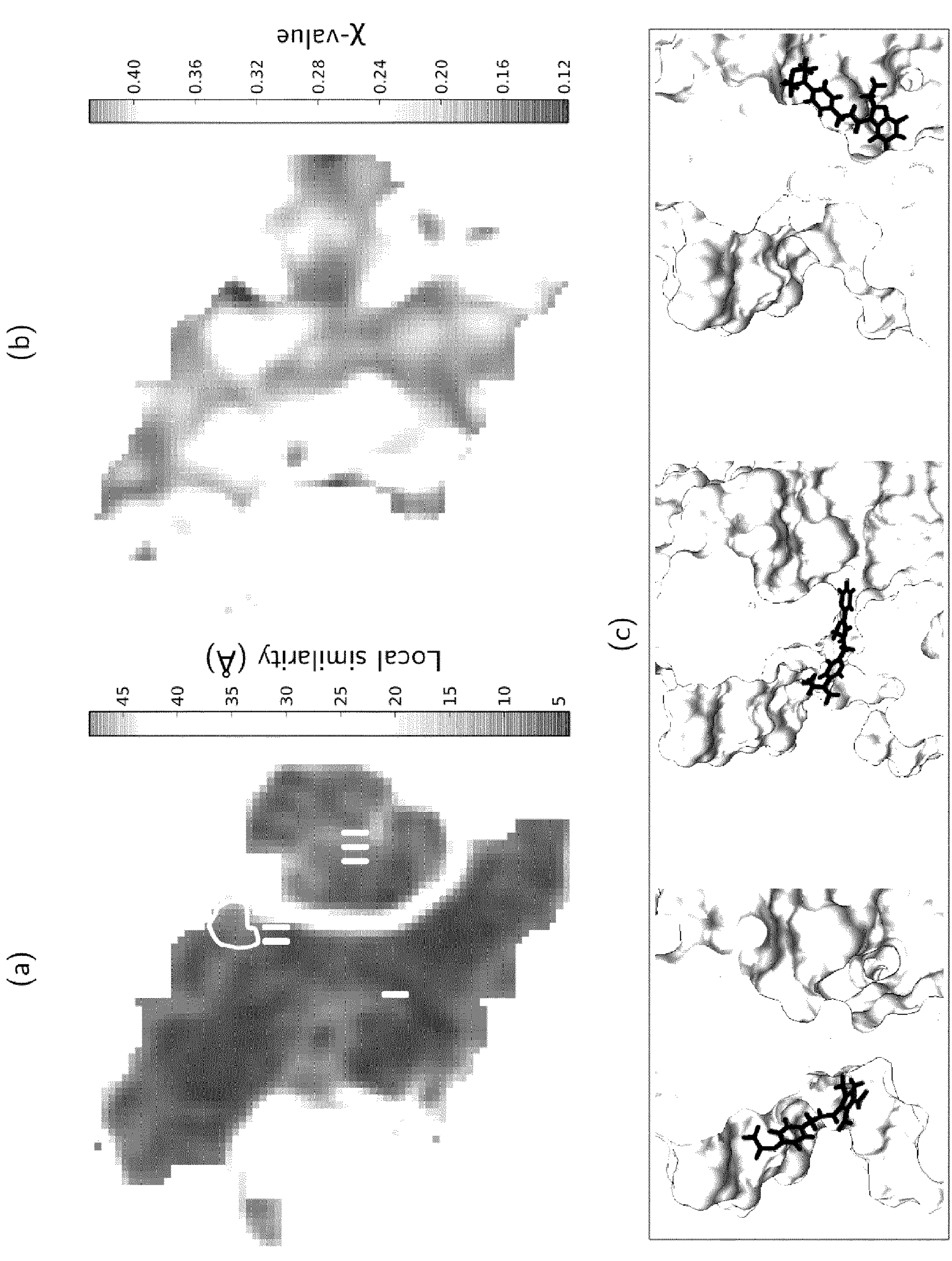
(a)
(b)
(c)
Local similarity (Å)
5
10
15
20
25
30
35
40
45
χ-value
0.12
0.16
0.20
0.24
0.28
0.32
0.36
0.40
I
II
III

FIG. 3

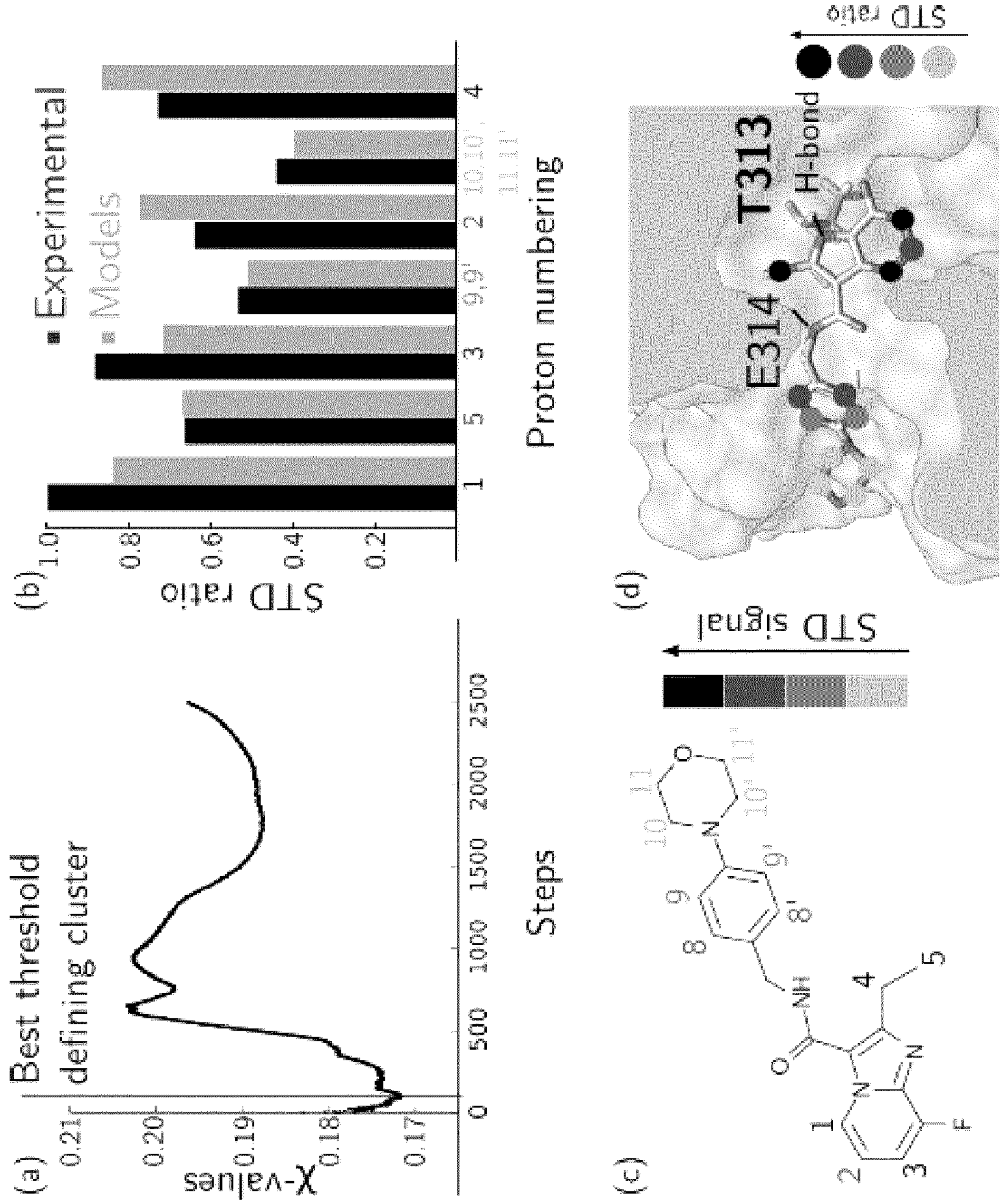
(a)
Best threshold
defining cluster
χ-values
0.21
0.20
0.19
0.18
0.17
0
500
1000
1500
2000
2500
Steps
(b)
STD ratio
1.0
0.8
0.6
0.4
0.2
Experimental
Models
1
5
3
9,9'
2
10,10', 11,11'
4
Proton numbering
(c)
STD signal
(d)
E314
T313
H-bond
STD ratio

FIG. 4

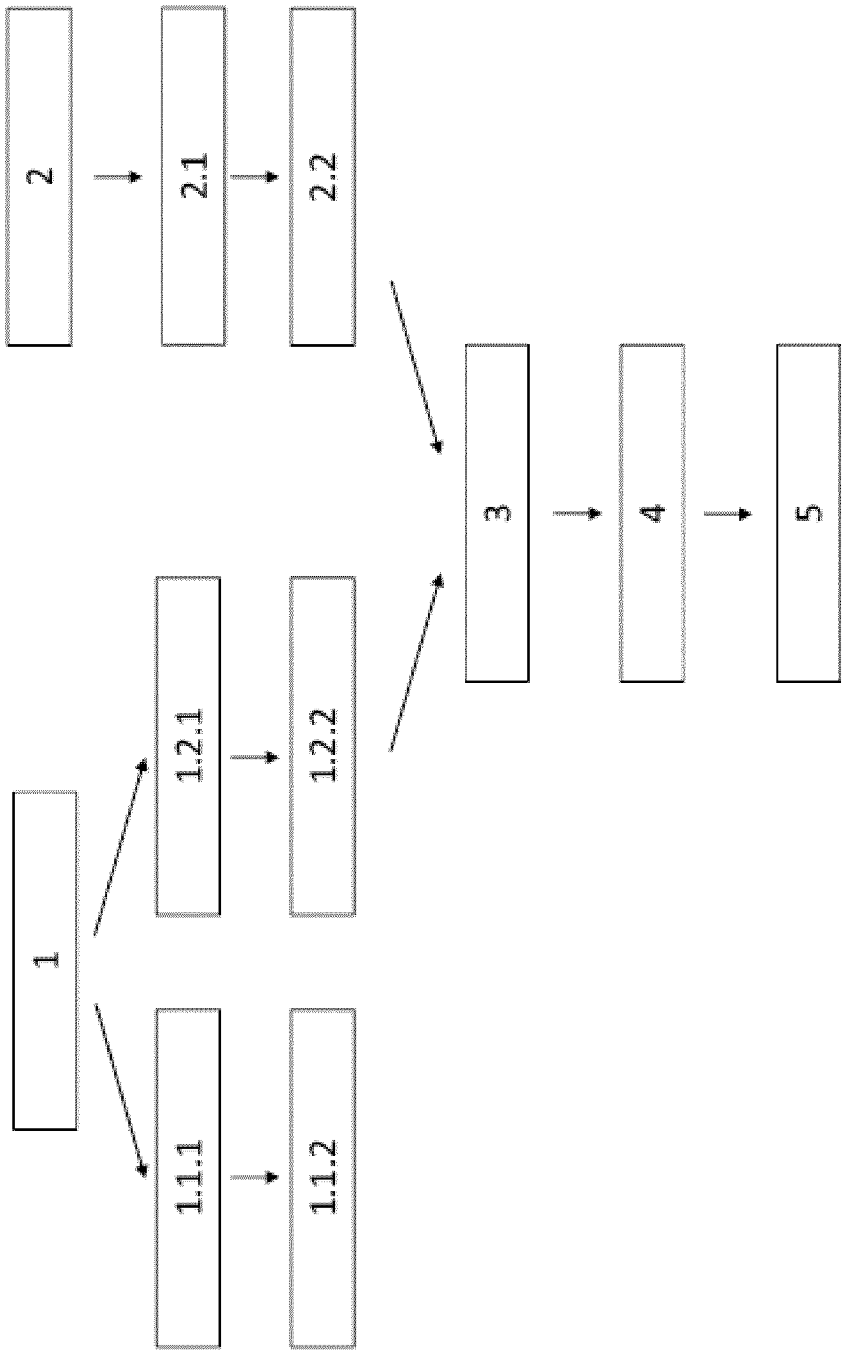


FIG. 5

Europäisches Patentamt
European Patent Office
Office européen des brevets

# EUROPEAN SEARCH REPORT

Application Number
EP 17 30 5915

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A,D | WO 2009/032727 A1 (UNIV FLORIDA [US]; WANG BING [US]; MERZ KENNETH MALCOLM [US]) 12 March 2009 (2009-03-12)<br>* the whole document *<br>----- | 1-15 | INV.<br>G06F19/16<br>G06F19/00 |
| A,D | BOUVIER G. ET AL: "Automatic clustering of docking poses in virtual screening process using self-organizing map", BIOINFORMATICS, vol. 26, no. 1, 12 November 2009 (2009-11-12), pages 53-60, XP055469535, GB<br>ISSN: 1367-4803, DOI: 10.1093/bioinformatics/btp623<br>* the whole document *<br>----- | 1-15 | |
| A,D | MARI S. ET AL: "1D saturation transfer difference NMR experiments on living cells: the DC-SIGN/oligomannose interaction", ANGEWANDTE CHEMIE INTERNATIONAL EDITION, VERLAG CHEMIE, vol. 44, no. 2, 27 December 2004 (2004-12-27), pages 296-298, XP002635153, ISSN: 1433-7851, DOI: 10.1002/ANIE.200461574<br>[retrieved on 2004-12-21]<br>* the whole document *<br>-----<br>-/-- | 1-15 | TECHNICAL FIELDS SEARCHED (IPC)<br>G06F |

The present search report has been drawn up for all claims

2

| Place of search | Date of completion of the search | Examiner |
|---|---|---|
| The Hague | 23 April 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 30 5915

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | DIGLES D. ET AL: "Self-Organizing Maps for In Silico Screening and Data Visualization", MOLECULAR INFORMATICS, vol. 30, no. 10, 2 September 2011 (2011-09-02), pages 838-846, XP055469550, ISSN: 1868-1743, DOI: 10.1002/minf.201100082 * the whole document * ----- | 1-15 | |
| A | MAYER M. ET AL: "Characterization of Ligand Binding by Saturation Transfer Difference NMR Spectroscopy", ANGEWANDTE CHEMIE INTERNATIONAL EDITION, vol. 38, no. 12, 14 June 1999 (1999-06-14) , pages 1784-1788, XP055469562, ISSN: 1433-7851, DOI: 10.1002/(SICI)1521-3773(19990614)38:12<1784::AID-ANIE1784>3.0.CO;2-Q * the whole document * ----- | 1-15 | TECHNICAL FIELDS SEARCHED (IPC) |
| A | ANGULO J. ET AL: "STD-NMR: application to transient interactions between biomolecules-a quantitative approach", EUROPEAN BIOPHYSICS JOURNAL ; WITH BIOPHYSICS LETTERS, SPRINGER, BERLIN, DE, vol. 40, no. 12, 24 September 2011 (2011-09-24), pages 1357-1369, XP019982885, ISSN: 1432-1017, DOI: 10.1007/S00249-011-0749-5 * the whole document * ----- | 1-15 | |
| | -/-- | | |

The present search report has been drawn up for all claims

| Place of search | Date of completion of the search | Examiner |
|---|---|---|
| The Hague | 23 April 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2
EPO FORM 1503 03.82 (P04C01)

Europäisches Patentamt
European Patent Office
Office européen des brevets

# EUROPEAN SEARCH REPORT

Application Number
EP 17 30 5915

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SOUSA S. F. ET AL: "Protein-Ligand Docking in the New Millennium - A Retrospective of 10 Years in the Field", CURRENT MEDICINAL CHEMISTRY, vol. 20, no. 18, 1 June 2013 (2013-06-01), pages 2296-2314, XP055469557, NL ISSN: 0929-8673, DOI: 10.2174/0929867311320180002 * the whole document * ----- | 1-15 | |

TECHNICAL FIELDS SEARCHED (IPC)

2

The present search report has been drawn up for all claims

| Place of search | Date of completion of the search | Examiner |
|---|---|---|
| The Hague | 23 April 2018 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 17 30 5915

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2018

| Patent document cited in search report | Publication date | Patent family member(s) | Publication date |
| --- | --- | --- | --- |
| WO 2009032727 A1 | 12-03-2009 | US 2010250217 A1 | 30-09-2010 |
| | | WO 2009032727 A1 | 12-03-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009032727 A
- WO 2011113606 A


### Non-patent literature cited in the description


- **DANIELA DIGLES ; GERHARD F. ECKER.** Self-Organizing Maps for In Silico Screening and Data Visualization. *Mol. Inf.,* 2011, vol. 30, 838-846
- **JUHA VESANTO.** SOM-based data visualization methods. *Intelligent Data Analysis,* 1999, vol. 3 (2), 111-126
- **BOUVIER, G. et al.** *Bioinformatics,* 2015, vol. 31, 1490
- **HOMANS, S. W.** *Angew Chem Int Ed Engl,* 2004, vol. 43, 290
- **PELLECCHIA, M. ; BERTINI, I. ; COWBURN, D. ; DALVIT, C. ; GIRALT, E. ; JAHNKE, W. ; JAMES, T. L. ; HOMANS, S. W. ; KESSLER, H. ; LUCHINAT, C.** *Nat Rev Drug Discov,* 2008, vol. 7 (7), 38
- **MEYER, B. ; PETERS, T.** *Angew Chem Int Ed Engl,* 2003, vol. 42, 864
- **CLAASEN, B. ; AXMANN, M. ; MEINECKE, R. ; MEYER, B.** *J Am Chem Soc,* 2005, vol. 127, 916
- **MARI, S. ; SERRANO-GOMEZ, D. ; CANADA, F. J. ; CORBI, A. L. ; JIMENEZ-BARBERO.** *J. Angew Chem Int Ed Engl,* 2004, vol. 44, 296
- **ASSADI-PORTER, F.M. ; TONELLI, M. ; MAILLET, E. et al.** *J Am Chem Soc,* 2008, vol. 130, 7212
- **BOUVIER, G. ; EVRARD-TODESCHI, N. ; GIRAULT, J. P ; BERTHO, G.** *Bioinformatics,* 2010, vol. 26, 53
- **PETHE, K. ; BIFANI, P. ; JANG, J. ; KANG, S. ; PARK, S. ; AHN, S. ; JIRICEK, J. ; JUNG, J. ; JEON, H. K. ; CECHETTO, J.** *Nat Med,* 2013, vol. 19, 1157
- **MATSOSO, L. G. ; KANA, B. D. ; CRELLIN, P. K. ; LEA-SMITH, D. J. ; PELOSI, A. ; POWELL, D. ; DAWES, S. S. ; RUBIN, H. ; COPPEL, R. L. ; MIZRAHI, V.** *J Bacteriol,* 2005, vol. 187, 6300
- **GEEN, H. ; FREEMAN, R.** *JMagn Reson,* 1991, vol. 93, 93
- **HWANG, T. L. ; SHAKA, A. J.** *J Magn Reson Ser A,* 1995, vol. 112, 275
- **KELLEY, L. A. ; MEZULIS, S. ; YATES, C. M. ; WASS, M. N. ; STERNBERG, M. J.** *Nat Protoc,* 2015, vol. 10, 845
- **ESSER, L. ; ELBERRY, M. ; ZHOU, F. ; YU, C. A. ; YU, L. ; XIA, D.** *J Biol Chem,* 2008, vol. 283, 2846
- **JONES, D. T.** *Bioinformatics,* 2007, vol. 23, 538
- **COMBS, S. A. ; DELUCA, S. L. ; DELUCA, S. H. ; LEMMON, G. H. ; NANNEMANN, D. P. ; NGUYEN, E. D. ; WILLIS, J. R. ; SHEEHAN, J. H. ; MEILER, J.** *Nat Protoc,* 2013, vol. 8, 1277
- **LANG, P. T. ; BROZELL, S. R. ; MUKHERJEE, S. ; PETTERSEN, E. F. ; MENG, E. C. ; THOMAS, V. ; RIZZO, R. C. ; CASE, D. A. ; JAMES, T. L. ; KUNTZ, I. D.** *RNA,* 2009, vol. 15, 1219
- **MANTSYZOV, A. B. ; BOUVIER, G. ; EVRARD-TODESCHI, N. ; BERTHO, G.** *Adv Appl Bioinform Chem,* 2012, vol. 5, 61
- **BOUVIER, G. ; DESDOUITS, N. ; FERBER, M. ; BLONDEL, A. ; NILGES, M.** *Bioinformatics,* 2015, vol. 31, 1490
- **JUHA VESANTO.** *Intelligent Data Analysis,* 1999, vol. 3 (2), 111-126